# EUROPEAN PATENT SPECIFICATION

(11) **EP 3 280 456 B1**
(45) Date of publication and mention of the grant of the patent: **03.03.2021**
(21) Application number: 16715309.7
(22) Date of filing: 08.04.2016
(51) Int. Cl.: A61K 51/12, A61K 38/18, A61K 38/20, A61K 38/28, C07K 5/02, A61P 3/10, A61P 5/48

(54) **NANOPARTICLE-BASED ANTIGEN SPECIFIC IMMUNOTHERAPY**
NANOPARTIKELBASIERTE ANTIGENSPEZIFISCHE IMMUNTHERAPIE
IMMUNOTHÉRAPIE SPÉCIFIQUE D'ANTIGÈNES À BASE DE NANOPARTICULES

(30) Priority: 10.04.2015 GB 201506112
(43) Date of publication of application: 14.02.2018
(73) Proprietor: Midatech Limited, Abingdon, Oxfordshire OX14 4RQ (GB); University College Cardiff Consultants Limited, Cardiff, South Glamorgan CF24 0DE (GB)
(72) Inventor: MCATEER, Martina, Abingdon Oxfordshire OX14 4RQ (GB); DAYAN, Colin Mark, Bristol Bristol BS2 8BX (GB); BIRCHALL, James Caradoc, Cardiff South Glamorgan CF3 2UT (GB)
(74) Representative: Mewburn Ellis LLP
(86) International application number: PCT/EP2016/057781
(87) International publication number: WO 2016/162495

(56) References cited:
- WO-A1-2005/073248
- WO-A1-2014/125256
- WO-A1-2014/135840
- WO-A2-2012/007950
- US-A1- 2015 099 698
- Shuhong Han ET AL: "Review Article Novel autoantigens in type 1 diabetes", Am J Transl Res, 1 January 2013 (2013-01-01), XP055278929, Retrieved from the Internet: URL:http://www.ncbi.nlm.nih.gov/pmc/articl es/PMC3665912/pdf/ajtr0005-0379.pdf
- T. DELONG ET AL: "Islet Amyloid Polypeptide Is a Target Antigen for Diabetogenic CD4+ T Cells", DIABETES, vol. 60, no. 9, 6 July 2011 (2011-07-06), pages 2325-2330, XP055278938, US ISSN: 0012-1797, DOI: 10.2337/db11-0288

### SEQUENCE LISTING

<110> Midatech Limited University College Cardiff Consultants Limited NanoPass Technologies Ltd. Leiden University Medical Center Institut National de la Sante et de la Recherche Medicale
<120> Nanoparticle - Based Antigen Specific Immunotherapy
<130> CSC/BP7190267
<150> GB 1506112.0
   <151> 2015-04-10
<160> 12
<170> PatentIn version 3.3
<210> 1
   <211> 18
   <212> PRT
   <213> Homo sapiens
<400> 1
   Gly Ser Leu Gln Pro Leu Ala Leu Glu Gly Ser Leu Gln Lys Arg Gly
   1               5                   10                  15
   Ile Val
<210> 2
   <211> 14
   <212> **PRT**
   <213> Homo sapiens
<400> 2
   Thr Val Tyr Gly Ala Phe Asp Pro Leu Leu Ala Val Ala Asp
   1               5                   10
<210> 3
   <211> 10
   <212> PRT
   <213> Mus musculus
<400> 3
   Tyr Val Arg Pro Leu Trp Val Arg Met Glu
   1 5 10
<210> 4
   <211> 14
   <212> PRT
   <213> Mus musculus
<400> 4
   Trp Ser Arg Met Asp Gln Leu Ala Lys Glu Leu Thr Ala Glu
   1 5 10
<210> 5
   <211> 15
   <212> PRT
   <213> Mus musculus
<400> 5
   Ser His Leu Val Glu Ala Leu Tyr Leu Val Ser Gly Glu Arg Gly
   1 5 10 15
<210> 6
   <211> 14
   <212> PRT
   <213> Homo sapiens
<400> 6
   Trp Ser Lys Met Asp Gln Leu Ala Lys Glu Leu Thr Ala Glu
   1 5 10
<210> 7
   <211> 110
   <212> PRT
   <213> Homo sapiens
<400> 7
<210> 8
   <211> 17
   <212> PRT
   <213> Artificial sequence
<220>
   <223> Murine peptide with linker at the N-terminus
<400> 8
   Ala Ala Tyr Trp Ser Arg Met Asp Gln Leu Ala Lys Glu Leu Thr Ala
   1               5                   10                  15
   Glu
<210> 9
   <211> 13
   <212> PRT
   <213> Artificial sequence
<220>
   <223> Murine peptide with linker at the N-terminus
<400> 9
   Ala Ala Tyr Tyr Val Arg Pro Leu Trp Val Arg Met Glu
   1               5                   10
<210> 10
   <211> 18
   <212> PRT
   <213> Artificial sequence
<220>
   <223> Murine peptide with linker at the N-terminus
<400> 10
   Ala Ala Tyr Ser His Leu Val Glu Ala Leu Tyr Leu Val Ser Gly Glu
   1               5                   10                  15
   Arg Gly
<210> 11
   <211> 17
   <212> PRT
   <213> Gallus gallus
<400> 11
   Ile Ser Gln Ala Val His Ala Ala His Ala Glu Ile Asn Glu Ala Gly
   1               5                   10                  15
   Arg
<210> 12
   <211> 12
   <212> PRT
   <213> Artificial sequence
<220>
   <223> Artifically created hybrid peptide
<400> 12
   Asp Leu Gln Thr Leu Ala Leu Trp Ser Arg Met Asp
   1               5                   10

## Description

### Field of the invention

The present invention relates to nanoparticles and tolerogenic vaccines comprising the nanoparticles, particularly for use in medicine, and includes use for treatment of certain auto-immune disorders such as diabetes mellitus type 1 ("type 1 diabetes" or "T1D"). Pharmaceutical compositions, processes for production of the nanoparticles and tolerogenic vaccines, and methods for their use are also disclosed.

### Background to the invention

The present invention is directed at compositions and products, and methods of making and administering such compositions and products, including for use in the treatment of mammals and particularly humans.

WO2006/037979 describes gold nanoparticles (GNPs) comprising adjuvants and antigens, such as tumour and pathogen antigens, and their use in a range of applications such as for the treatment of cancer and infectious diseases. Also disclosed are immunogenic structures based on nanoparticles or antibodies with carbohydrate ligands, and their use for therapeutic and prophylactic purposes, and for the isolation and detection of antibodies directed against the carbohydrate structures.

WO2013/034741 and WO2013/034726 describe nanoparticles having an epitopic peptide bound via a linker and which find use as vaccines, e.g., in the prophylactic or therapeutic treatment of a tumour in a mammalian subject.

WO2011/154711 describes glycated gold nanoparticles that act as carriers for delivery of peptides such as insulin.

WO 2014/125256 discloses compositions for use in targeting biologically active agents to the central nervous system comprising: i) a core comprising a metal and/or a semiconductor; and (ii) a corona comprising a plurality of ligands covalently linked to the core, wherein said ligands comprise a carbohydrate, insulin and/or a glutathione; and (b) the at least one agent to be delivered to the CNS.

Antigen-specific immunotherapy has been proposed as a treatment strategy for autoimmune diseases, including type 1 diabetes (Peakman and Dayan, Immunology, 2001, Vol. 104(4), pp. 361-366). Proinsulin peptide C19-A3 has been employed in a phase I human study to evaluate safety and mechanistic outcomes for immunotherapy of type 1 diabetes (Thrower et al., Clin. Exp. Immunol., 2009, Vol. 155(2), pp. 156-165). Current approaches to improving glycaemic control in type 1 diabetes are centred on increasingly complex insulin delivery systems. However, less than 30% of patients can achieve target levels of glucose control with this approach even in a clinical trial setting and many patients are either unable or unwilling to make the personal commitment required. By contrast, preservation of even small amounts of endogenous insulin production, has been shown to improve glycaemic control, reduce hypoglycaemia, improve quality of life and reduce long-term complications. Importantly, glycaemic control in the presence of endogenous beta cell function is not demanding and hence would be effective across the full spectrum of individuals. Antigen specific immunotherapy (ASI) is the preferred approach to beta cell preservation since this avoids the risks of immunosuppression. The Enhanced Epidermal Antigen Specific Immunotherapy (EE-ASI) website available at www.ee-asi.eu proposes an approach in which a beta cell target T cell epitope (proinsulin C19-A3) will be combined with the tolerogenic cytokine IL-10 and targeted to antigen presenting cells via gold nanoparticles and delivery into the very superficial layers of the skin using microneedles.

Attempts at ASI to date although successful in preclinical models have had limited efficacy in humans. There is therefore an urgent need for the development of novel approaches to deliver effective ASI. The present invention addresses these and other needs.

### Brief Description of the Invention

Broadly, the present invention relates to the nanoparticle-based delivery of an autoantigen peptide (e.g. the C19-A3 proinsulin peptide) and a tolerogenic cytokine (e.g. IL-10) to provide targeted antigen-specific immunotherapy, wherein the nanoparticles are as defined in claims 1 to 4 or claim 8. The present inventors have surprisingly found that mixed glycan- and glutathione-coronated gold nanoparticles exhibit efficient binding of C19-A3 peptide and IL-10 cytokine payloads, respectively, and that such nanoparticle constructs deliver their payload via intradermal administration and induce antigen-specific tolerance and widespread promotion of a pro-tolerogenic phenotype in dendritic cells as well as reducing production of inflammatory cytokines. Indeed, the present inventors have surprisingly found that the autoantigen-loaded mixed glycan- and glutathione-coronated gold nanoparticles are able to bind recombinant human IL-10 electrostatically at binding efficiency of up to 100% (see Example 9 herein). Significantly, as reported herein, the nanoparticle-based delivery of autoantigen peptide payload following dermal microinjection was found to reach more distant lymph nodes, including pancreatic lymph nodes, a site relevant to the beta cell target for type 1 diabetes immunotherapy.

Accordingly, in a first aspect the present invention provides a nanoparticle comprising:
a core comprising metal atoms;
a corona comprising a plurality of ligands covalently linked to the core, wherein said plurality of ligands comprises:
   at least one carbohydrate ligand;
   at least one glutathione ligand; and
   at least one autoantigen peptide ligand
wherein the plurality of ligands are in the following proportions by number:
(i) as determined by the input proportions of the ligand reactants during nanoparticle synthesis:

| | |
|---|---|
| said autoantigen peptide ligand: | 0.1 - 10% |
| said carbohydrate ligand: | 1 - 10% |
| said glutathione ligand: | 80 - 98.9%; |

and/or
(ii) as determined by NMR carried out on the nanoparticle:

| | |
|---|---|
| said autoantigen peptide ligand: | 2 - 75% |
| said carbohydrate ligand: | 4 - 35% |
| said glutathione ligand: | 20 - 88% |

and wherein said autoantigen peptide ligand comprises a peptide selected from the group consisting of:
proinsulin peptide C19-A3 having the amino acid sequence GSLQPLALEGSLQKRGIV (SEQ ID NO: 1);
human proinsulin protein having the amino acid sequence or
a variant thereof differing by addition, substitution or deletion of not more than 5 amino acids;
Glutamic acid decarboxylase (GAD) (339-352) having the amino acid sequence TVYGAFDPLLAVAD (SEQ ID NO: 2);
BDC-2.5 mimotope peptide having the amino acid sequence YVRPLWVRME (SEQ ID NO: 3);
WE-14 peptide from chromogranin A 342-355 having the amino acid sequence WSRMDQLAKELTAE (SEQ ID NO: 4);
Insulin B (9-23) having the amino acid sequence SHLVEALYLVSGERG (SEQ ID NO: 5); and
a HIB peptide having the amino acid sequence DLQTLALWSRMD (SEQ ID NO: 12),
or a hybrid peptide comprising two or more of said peptides.

In some cases the carbohydrate of said carbohydrate ligand is a monosaccharide. In particular, the carbohydrate ligand may comprise glucose or mannose.

In some cases the ligands may be 3% autoantigen peptide (e.g. C19-A3 peptide), 5% carbohydrate ligand (e.g. glucose) and 92% glutathione (as determined by input proportions of the ligand reactants during nanoparticle synthesis).

In some cases in accordance with this and other aspects of the invention the plurality of ligands are selected as follows and are in the following proportions by number (as determined by input proportions of the ligand reactants during nanoparticle synthesis):
said autoantigen peptide comprises C19-A3 peptide having the amino acid sequence GSLQPLALEGSLQKRGIV (SEQ ID NO: 1) and is present at 2 - 4%;
said carbohydrate ligand comprises glucose and is present at 4 - 6%; and
said glutathione ligand is present at 90 - 94%.

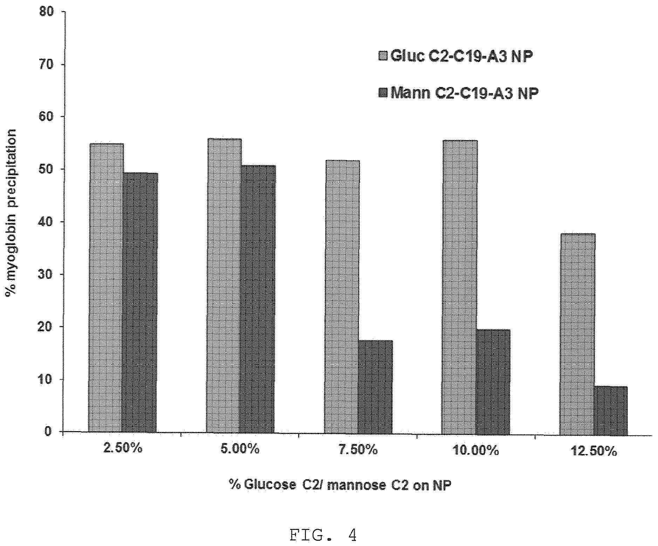

As described in Example 7 herein, the ligand percentages may differ depending on the method of determination (e.g. reaction input vs. post-synthesis characterisation, such as by NMR and/or HPLC of the final nanoparticles). Accordingly, the same nanoparticles may also be described in terms of percentages determined by NMR carried out on the nanoparticle
The proportions (by NMR) defined in claim 1 correspond to the proportions (by reactant input) that were found to exhibit optimal IL-10 electrostatic binding - see Figure 4 and Example 2. Indeed 5% glucose-C2 (by input percentage) displayed excellent myoglobin binding (proxy for IL-10 binding) and corresponds to around 27% glucose-C2 (by NMR).

In certain cases, the plurality of ligands may be in the following proportions by number (as determined by NMR carried out on the nanoparticles):

| | |
|---|---|
| said autoantigen peptide ligand: | 4 - 15% |
| said carbohydrate ligand: | 12 - 35% |
| said glutathione ligand: | 50 - 88%. |

The same nanoparticles may also be described in terms of the number of ligands covalently bound to the nanoparticle core. In particular, the plurality of ligands may be in the following amounts by number (as determined by NMR carried out on the nanoparticles):

| | |
|---|---|
| said autoantigen peptide ligand: | 1-30 |
| said carbohydrate ligand: | 2-14 |
| said glutathione ligand: | 10-35, |

wherein the total number of covalently bound ligands is between 40 and 50 (e.g. 44 ligands) per nanoparticle core.

In certain embodiments, the plurality of ligands may be in the following amounts by number (as determined by NMR carried out on the nanoparticles):

| | |
|---|---|
| said autoantigen peptide ligand: | 2-6 |
| said carbohydrate ligand: | 6-14 |
| said glutathione ligand: | 25-35, |

wherein the total number of covalently bound ligands is between 40 and 50 (e.g. 44 ligands) per nanoparticle core.

In some cases in accordance with this and other aspects of the invention the autoantigen peptide ligand is covalently bound to the core via a linker comprising:

-S(CH₂)₂-CONH-

or

-S(CH₂)₂-CONH-AlaAlaTyr- .

The sulphur atom of the linker being bound to the surface of the nanoparticle core and the N-terminus of the autoantigen peptide being bound to the other end of the linker (i.e. the end not bound to the nanoparticle core).

In some cases in accordance with this and other aspects of the invention the carbohydrate ligand is covalently bound to the core via a linker comprising -S(CH₂)ₙ-, wherein n is 1 to 11.

In some cases in accordance with this and other aspects of the invention the nanoparticle further comprises an anti-inflammatory cytokine that is non-covalently bound to the corona of the nanoparticle. In particular cases the anti-inflammatory cytokine may be selected from the group consisting of: IL-10, (IL)-1 receptor antagonist, IL-4, IL-5, IL-11, IL-13, TGF-beta 1 and TGF-beta 2. Preferably, the anti-inflammatory cytokine is human IL-10. The present inventors have surprisingly found that an autoantigen peptide carrying nanoparticle of the invention is able to additionally bind non-covalently (e.g. electrostatically) IL-10 so as to produce a "dual cargo" nanoparticle with both a self-antigen and a tolerogenic cytokine for delivery to antigen presenting cells (APCs) such as dendritic cells (DCs) and can thereby be expected to provide therapeutic benefit in the form of antigen specific immunotherapy for an autoimmune disorder such as diabetes mellitus type 1.

In some cases in accordance with this and other aspects of the invention the autoantigen peptide ligand comprises C19-A3 peptide and the anti-inflammatory cytokine comprises human IL-10, wherein the IL-10 polypeptide and C19-A3 peptide are present in a ratio between 1:1 and 1:10 IL-10:C19-A3 (e.g. between 1:3 and 1:8 IL-10:C19-A3), but IL-10 may also be present in lesser ratios such that it is not present in all particles, but makes up a final administered ratio of IL-10:peptide of 1:1000 to 1:100, by number (i.e. molar ratio).

In a second aspect the present invention provides a nanoparticle comprising:
a core comprising metal atoms;
a corona comprising a plurality of ligands covalently linked to the core, wherein said plurality of ligands comprises at least one carbohydrate ligand and at least one glutathione ligand; and
at least one IL-10 polypeptide non-covalently bound to the corona.

The present inventors have surprisingly found that a nanoparticle as defined herein is able to non-covalently (e.g. electrostatically) bind IL-10 and, wherein injected intradermally, deliver the IL-10 to distant lymph nodes in order to contribute to a tolerogenic phenotype that is more widespread than that achieved by free IL-10 administered in the absence of a nanoparticle of the invention. Indeed, nanoparticle-bound IL-10 is shown herein to induce de *novo* production of IL-10 by dendritic cells (e.g. as measured by IL-10 mRNA expression) and to result in a suppressive T cell response to a target antigen - see Example 6 herein.

In some cases in accordance with this and other aspects of the invention the carbohydrate of said carbohydrate ligand is a monosaccharide. In particular, the carbohydrate ligand may comprise glucose or mannose.

In some cases said ligands are in the following proportions by number (as determined by reactant input proportions for nanoparticle synthesis):

| | |
|---|---|
| said carbohydrate ligand: | 1 - 10% |
| said glutathione ligand: | 90 - 99%. |

The same nanoparticles may be described in terms of ligand percentages determined by analysis of the finished nanoparticles (e.g. by NMR and/or HPLC). In particular, the ligands may be in the following proportions by number (as determined by NMR on the nanoparticles):

| | |
|---|---|
| said carbohydrate ligand: | 2 - 30% |
| said glutathione ligand: | 70 - 98%. |

In some cases the carbohydrate ligand is covalently bound to the core via a linker comprising -S(CH₂)ₙ-, wherein n is 1 to 11.

In some cases in accordance with this and other aspects of the invention there are between 1 and 5 (e.g. between 1 and 3) IL-10 polypeptides per nanoparticle.

In some cases in accordance with the first, second and other aspects of the invention there are at least 20, at least 30, at least 40 or at least 50 ligands per nanoparticle.

In some cases in accordance with the first, second and other aspects of the invention the diameter of the core of the nanoparticle is in the range 1 nm to 5 nm.

In some cases in accordance with the first, second and other aspects of the invention the diameter of the nanoparticle including its ligands is in the range 2 nm to 50 nm.

In some cases in accordance with the first, second and other aspects of the invention the core comprises a metal selected from the group consisting of: Au, Ag, Cu, Pt, Pd, Fe, Co, Gd and Zn, or any combination thereof. In particular, the core may be of gold atoms.

In a third aspect the present invention provides a pharmaceutical composition comprising a plurality of nanoparticles of the first aspect of the invention and/or of the second aspect of the invention, and a pharmaceutically acceptable carrier, salt and/or diluent. In particular, the composition of the third aspect of the invention may be in the form of a vaccine, e.g. a vaccine for the treatment of an autoimmune disease such as type 1 diabetes.

In some cases the plurality of nanoparticles comprises at least one nanoparticle of the first aspect of the invention, wherein the composition further comprises IL-10 either free or associated with said nanoparticles. For example, a composition of autoantigen peptide-carrying nanoparticles of the first aspect of the invention may be admixed with free IL-10 or may be in the form of an article of manufacture comprising a first container comprising autoantigen peptide-carrying nanoparticles or composition thereof and a second container comprising free IL-10 or composition thereof.

In some cases the plurality of nanoparticles comprises at least one nanoparticle of the first aspect of the invention and at least one nanoparticle of the second aspect of the invention. In this way autoantigen peptide-carrying nanoparticles and IL-10 carrying nanoparticles may advantageously be provided in suitable ratios for simultaneous or sequential delivery. For example, the composition may be in the form of two injectable doses.

In some cases the plurality of nanoparticles comprises C19-A3 peptide-carrying nanoparticles and IL-10-carrying nanoparticles, wherein said C19-A3 peptide-carrying nanoparticles and IL-10-carrying nanoparticles are present at a ratio such that the ratio of C19-A3 peptide and IL-10 polypeptide is between 1:1 and 1:10000, such as between 1:1 and 1:1000, between 1:1 and 1:100, or between 1:1 and 1:20 IL-10:C19-A3, by number.

In some cases in accordance with the third aspect of the invention the pharmaceutical composition may be in the form of a vaccine (e.g. an injectable vaccine).

In a fourth aspect the present invention provides a nanoparticle of the first or second aspect of the invention or a pharmaceutical composition of the third aspect of the invention for use in medicine.

In a fifth aspect the present invention provides a nanoparticle for use in antigen specific immunotherapy of an autoimmune disorder in a mammalian subject wherein the nanoparticle comprises: a core comprising metal atoms; a corona comprising a plurality of ligands covalently linked to the core, wherein said plurality of ligands comprises: at least one carbohydrate ligand; at least one glutathione ligand; and at least one autoantigen peptide ligand. In the nanoparticles provided by the fifth aspect of the present invention, the plurality of ligands are in the following proportions by number:
(i) as determined by the input proportions of the ligand reactants during nanoparticle synthesis:

| | |
|---|---|
| said autoantigen peptide ligand: | 0.1 - 10% |
| said carbohydrate ligand: | 1 - 10% |
| said glutathione ligand: | 80 - 98.9%; |

and/or
(ii) as determined by NMR carried out on the nanoparticle:

| | |
|---|---|
| said autoantigen peptide ligand: | 2 - 75% |
| said carbohydrate ligand: | 4 - 35% |
| said glutathione ligand: | 20 - 88% |

In the fifth aspect of the present invention, the nanoparticle:
(a) delivers the autoantigen peptide to antigen presenting cells (APCs); and/or
(b) delivers the autoantigen peptide to draining lymph nodes via migratory DCs; and/or
(c) delivers the autoantigen peptide to pancreatic lymph nodes.

The autoimmune disorder is generally selected in conjunction with the appropriate autoantigen peptide. In particular, the following combinations of autoimmune disorder and autoantigen peptide(s) are specifically contemplated herein:
type 1 diabetes (e.g. insulin or proinsulin or fragment thereof (e.g. proinsulin C19-A3), glutamic acid decarboxylase (GAD), IA2, islet cell antigens, chromogranin A (e.g. chromogranin A342-355 or synthetic peptide mimicking chromogranin A342-355 peptide, the BDC2.5 mimotope), zinc transporter 8(ZnT8/Slc30A8)), a hybrid peptide having the amino acid sequence DLQTLALWSRMD (SEQ ID NO: 12);
multiple sclerosis (MS) (e.g. myelin basic protein (MBP));
primary biliary cirrhosis (PBC) (e.g. pyruvate dehydrogenase complex (PDC-E2);
myasthenia gravis (e.g. nicotinic acetylcholine receptor (nAChR);
rheumatoid arthritis (RA) (e.g. collagen type II); and autoimmune thyroid disease (e.g. thyroid peroxidase, thyroglobulin and TSH receptor).

In a sixth aspect the present invention provides a nanoparticle of the first or second aspect of the invention or a pharmaceutical composition of the third aspect of the invention for use in antigen specific immunotherapy of an autoimmune disorder in a mammalian subject.

In particularly preferred cases in accordance with the fifth or sixth aspect of the present invention the autoimmune disorder may be diabetes mellitus type 1.

In some cases in accordance with the fifth or sixth aspect of the present invention, there is provided a nanoparticle comprising:
a core comprising gold atoms, said core having a diameter in the range 1 nm to 5 nm;
a corona comprising a plurality of ligands covalently linked to the core, wherein said plurality of ligands comprises, in the following proportions by number, as determined by NMR:
   (i) autoantigen peptide ligands comprising proinsulin peptide C19-A3 having the amino acid sequence GSLQPLALEGSLQKRGIV (SEQ ID NO: 1): 2-75%;
   (ii) monosaccharide ligands: 4-35%; and
   (iii) glutathione ligands: 20-88%,
   for use in antigen specific immunotherapy of diabetes mellitus type 1 in a mammalian subject.

In some cases in accordance with the fifth or sixth aspect of the invention the IL-10-carrying nanoparticles or composition thereof and proinsulin C19-A3 peptide-carrying nanoparticles or composition thereof are delivered simultaneously or sequentially. For example, said IL-10-carrying nanoparticles or composition thereof may be delivered as a first dose and said proinsulin C19-A3 peptide-carrying nanoparticles or composition thereof may be delivered as a second dose, wherein said first and second doses are delivered within 24 hours of one another (e.g. within 12 hours, 8 hours, 4 hours, 2 hours, 1 hour, 30 minutes, 10 minutes or 5 minutes of one another). In some cases the IL-10-carrying nanoparticles or composition thereof are delivered prior to the proinsulin C19-A3 peptide-carrying nanoparticles or composition thereof. However, the converse wherein the proinsulin C19-A3 peptide-carrying nanoparticles or composition thereof are delivered prior to the IL-10-carrying nanoparticles or composition thereof is specifically contemplated.

In some cases in accordance with the sixth aspect of the present invention, the nanoparticle may be as defined in accordance with the first aspect of the invention wherein the nanoparticle is a "dual cargo" nanoparticle comprising both a autoantigen peptide and an anti-inflammatory cytokine.

In some cases in accordance with the fifth or sixth aspect of the present invention the nanoparticle or composition thereof is for:
inducing a tolerogenic phenotype in a dendritic cell (DC);
enhancing production of tolerogenic cytokines;
enhancing production of regulatory T cells; and/or
preserving pancreatic beta cell function,
in said subject.

In some cases in accordance with the fifth or sixth aspect of the present invention the nanoparticle or composition thereof is delivered via a dermal route of administration.

In some cases in accordance with the fifth or sixth aspect of the present invention the nanoparticle or composition thereof is delivered via microneedle injection. In particular, the microneedle injection may be to the superficial layers of the skin.

In some cases in accordance with the sixth aspect of the present invention the nanoparticle or composition thereof delivers the autoantigen peptide and/or the anti-inflammatory cytokine to CD207+ Langerhans antigen presenting cells (APCs).

In some cases in accordance with the sixth aspect of the present invention the nanoparticle or composition thereof delivers the autoantigen peptide and/or the anti-inflammatory cytokine to draining lymph nodes via migratory DCs.

In some cases in accordance with the sixth aspect of the present invention the nanoparticle or composition thereof delivers the autoantigen peptide and/or the anti-inflammatory cytokine to pancreatic lymph nodes. It is considered particularly advantageous that the nanoparticles of the present invention, when administered intradermally, exhibit delivery of autoantigen peptide to pancreatic lymph nodes as delivery to pancreatic lymph nodes is expected to be of significant benefit for the treatment of type 1 diabetes by antigen specific immunotherapy. Indeed the superior ability of nanoparticle-carried autoantigen peptide and/or IL-10 to reach the target organ following delivery at the superficial layers of the skin in comparison with injection of free antigen peptide and/or free IL-10 underscores the benefit of the nanoparticle cargo approach taught herein.

In some cases in accordance with the fifth or sixth aspect of the present invention the subject has been diagnosed with, or is at risk of developing, diabetes mellitus type 1 or pre-diabetes. In particular, the subject may have been tested positive for one or more autoantibodies associated with diabetes mellitus type 1.

In a seventh aspect the present invention provides a method for producing a nanoparticle of the first or second aspects of the invention, comprising:
providing at least one thiol-derivatised autoantigen peptide, at least one glutathione, at least one thiol-derivatised carbohydrate, at least one salt of a core-forming metal, and a reducing agent;
reacting the at least one thiol-derivatised autoantigen peptide, the at least one glutathione, the at least one thiol-derivatised carbohydrate, the at least one salt of a core-forming metal, and the reducing agent so that during self-assembly of the nanoparticle, the nanoparticle core attaches the autoantigen peptide, the glutathione and the carbohydrate via their respective linkers. The salt may be a gold salt. The reducing agent may be sodium borohydride. The thiol-derivatised carbohydrate may be thioC2-glucose or thioC2-mannose. The thiol-derivatised autoantigen peptide may be thioproprionic acid-amide-C19-A3 peptide.

In some cases said reacting is performed in an aqueous solution of 5 - 75% methanol (w/v) or 100% water. In particular, 10% methanol or 100% water reactions were found to provide superior proinsulin C19-A3 peptide incorporation.

In some cases the method further comprises a purification step (e.g. centrifugation and/or dialysis, and filtration) to remove unreacted free reagents not covalently bound to the nanoparticle. In some cases, the method further comprises a step of sterilizing the produced nanoparticles and/or a composition comprising the produced nanoparticles, e.g. in order to render the nanoparticles and/or composition suitable for clinical use.

The method may further comprise formulating the nanoparticle into a composition with at least one pharmaceutically acceptable carrier, salt or diluent.

In some cases the method further comprises the step of bringing the nanoparticle into contact with at least one anti-inflammatory cytokine under conditions which allow the anti-inflammatory cytokine to bind non-covalently to the corona of the nanoparticle. For example, the anti-inflammatory cytokine may be IL-10.

Disclosed herein is a method of treatment of a mammalian subject having an autoimmune disorder, the method comprising administering to the subject a therapeutically effective amount of a nanoparticle of the first or second aspect of the invention or a pharmaceutical composition of the third aspect of the invention.

In some cases said autoimmune disorder is diabetes mellitus type 1.

In some cases IL-10-carrying nanoparticles or composition thereof and proinsulin C19-A3 peptide-carrying nanoparticles or composition thereof are delivered simultaneously or sequentially to the subject.

In some cases said IL-10-carrying nanoparticles or composition thereof are delivered as a first dose and proinsulin C19-A3 peptide-carrying nanoparticles or composition thereof are delivered as a second dose, and wherein said first and second doses are delivered within 24 hours of one another (e.g. within 12 hours, 8 hours, 4 hours, 2 hours, 1 hour, 30 minutes, 10 minutes or 5 minutes of one another).

In some cases the IL-10-carrying nanoparticles or composition thereof are delivered prior to the proinsulin C19-A3 peptide-carrying nanoparticles or composition thereof. However, the converse wherein the proinsulin C19-A3 peptide-carrying nanoparticles or composition thereof are delivered prior to the IL-10-carrying nanoparticles or composition thereof is specifically contemplated.

In some cases the nanoparticle is of the first aspect of the invention wherein the nanoparticle is a "dual cargo" nanoparticle comprising both an autoantigen peptide and an anti-inflammatory cytokine.

In some cases the nanoparticle or composition thereof:
induces a tolerogenic phenotype in a dendritic cell (DC);
enhances production of tolerogenic cytokines; enhancing production of regulatory T cells and/or
preserves pancreatic beta cell function,
in said subject.

In some cases the nanoparticle or composition thereof is delivered via a dermal route of administration.

In some cases the nanoparticle or composition thereof is delivered via single or multiple (e.g. 1, 2, 3, 4, or 5 needles per syringe) microneedle injection. For example the method may comprise a microneedle injection to the superficial layers of the skin.

In accordance with all aspects of the present invention the nanoparticles and methods for their production may be sterile. However, it is specifically contemplated herein that the production of the nanoparticles includes one or more sterilization steps.

In accordance with the present invention, particularly the fourth, fifth, and sixth aspects thereof, the subject may be a human, a companion animal (e.g. a dog or cat), a laboratory animal (e.g. a mouse, rat, rabbit, pig or non-human primate), a domestic or farm animal (e.g. a pig, cow, horse or sheep). Preferably, the subject is a human.

The present invention includes the combination of the aspects and preferred features described, except where such a combination is clearly impermissible or is stated to be expressly avoided. These and further aspects and embodiments of the invention are described in further detail below and with reference to the accompanying examples and figures.

### Brief Description of the figures

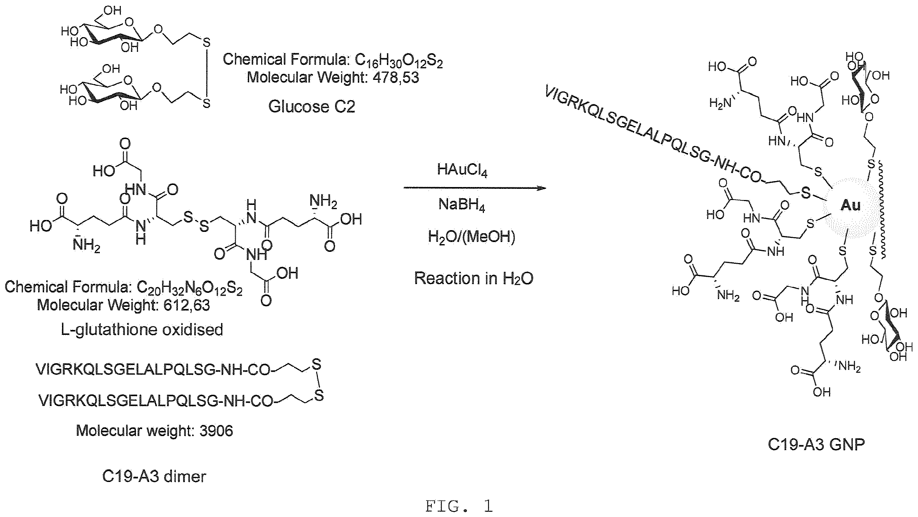

**Figure 1** shows the synthesis strategy employed for covalently binding proinsulin C19-A3 peptides to gold nanoparticles.

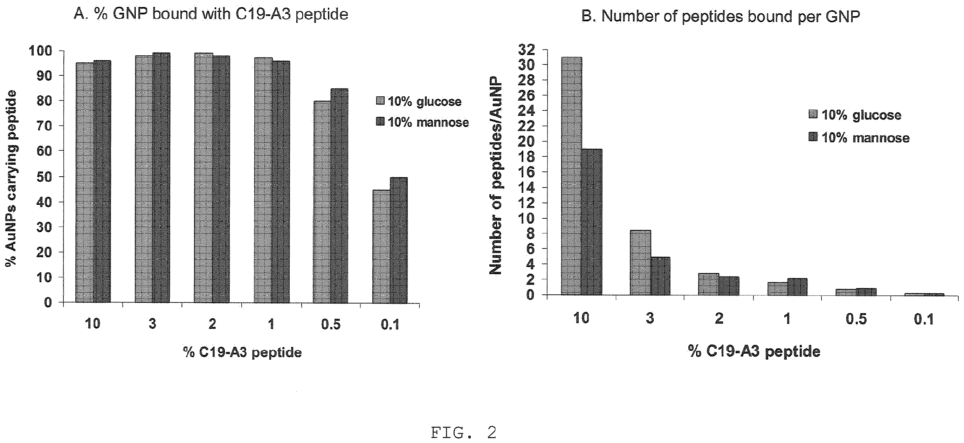

**Figure 2** shows A. the % of gold nanoparticles bound with proinsulin C19-A3 peptide; and B. the number of peptides bound per gold nanoparticle when nanoparticles are passivated with either 10% glucose C2 or 10% mannose C2.

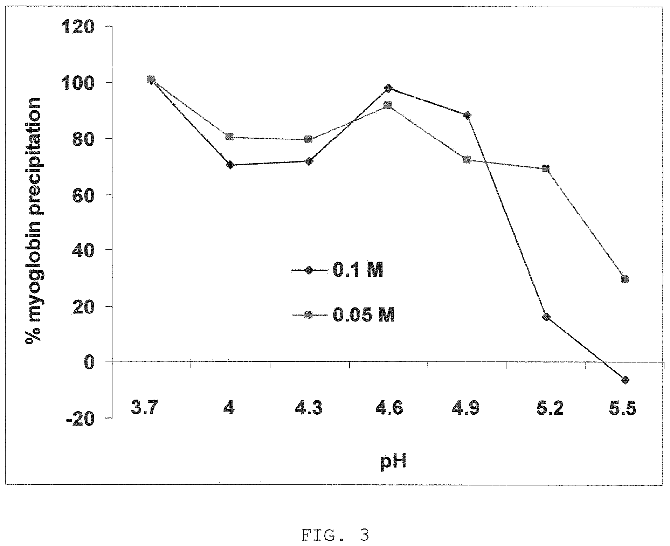

**Figure 3** shows the degree of electrostatic binding of IL-10 surrogate myoglobin to proinsulin C19-A3 peptide-GNPs at varying pH and myoglobin concentration.
**Figure 4** shows myoglobin binding to 1% proinsulin C19-A3 GNPs with different amounts of glucose C2 or mannose C2.

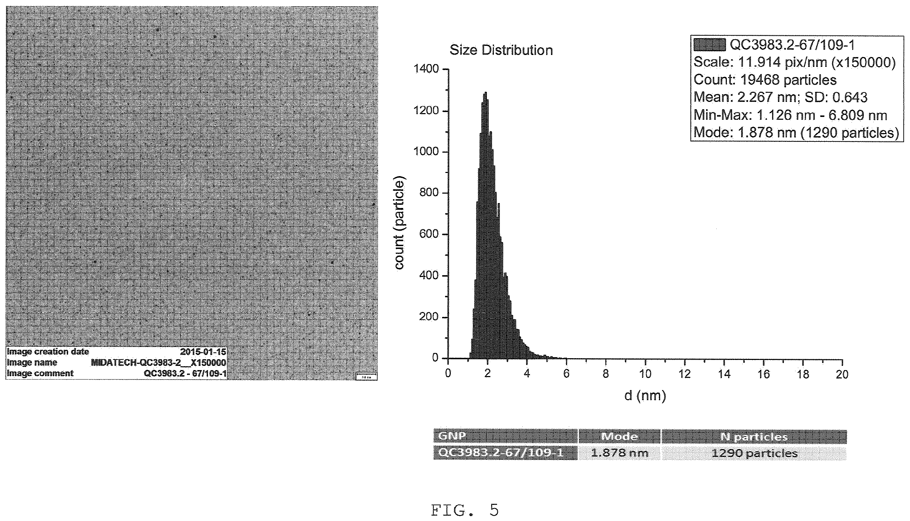

**Figure 5** shows TEM size analysis of 3% proinsulin C19-A3 GNPs manufactured for in vivo toxicology study.

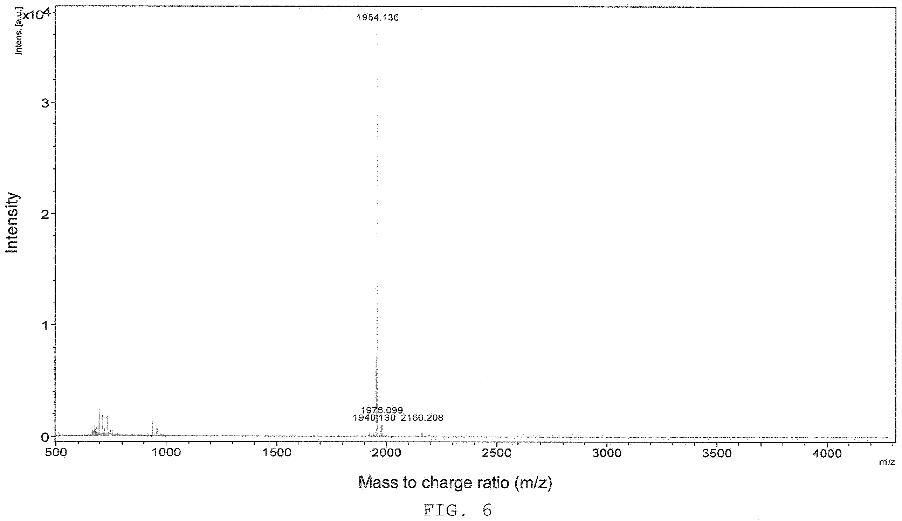

**Figure 6** shows MALDI-TOF mass spectrometry of proinsulin C19-A3 GNPs, and confirms that the proinsulin C19-A3 peptide is intact.

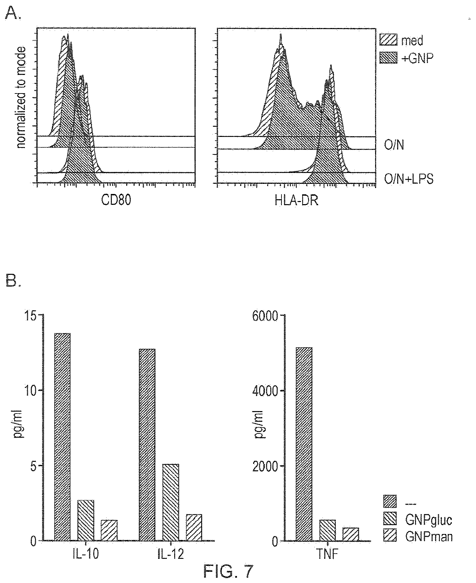

**Figure 7** shows that incubation of immature DCs with C19-A3 GNPs does not change DC phenotype but inhibits cytokine production. (A) Immature DCs (iDCs) were incubated with proinsulin C19-A3 coupled GNPs (red histograms) or without GNPs (grey histograms), for 4hr. Thereafter, GNPs were washed and cells were left overnight (O/N) either alone (light grey and red histograms - upper two rows) or in the presence of 100ng/ml LPS (dark grey and red histograms - lower two rows). Overnight maturation of DCs was measured as increase of CD80 and HLA-DR expression using FACS. (B) iDCs were incubated with proinsulin C19-A3 GNPs coupled to glucose (GNPgluc - red bars), with proinsulin C19-A3 GNPs coupled to mannose (GNPman - orange bars) or left untreated (light grey bars), for 4hrs, after which cells were washed and subsequently stimulated with 100ng/ml LPS and left overnight. Supernatants were collected the next day and cytokine (IL-10, IL-12 and TNF-α) release by DCs was measured using Luminex. Data are representative of three independent experiments (n=3).

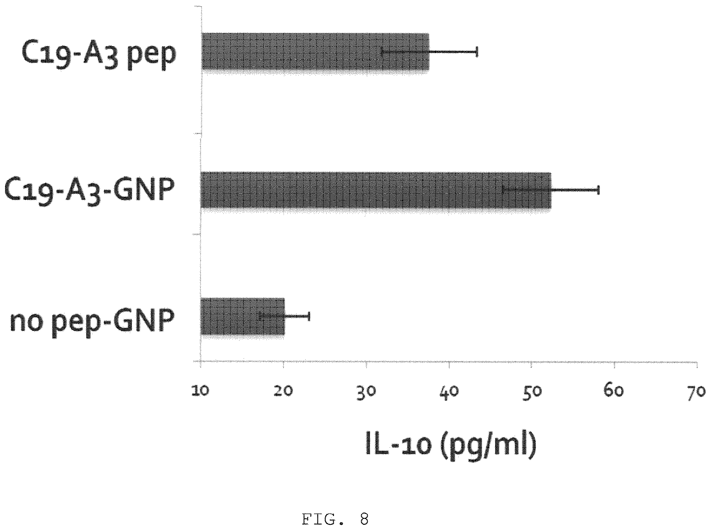

**Figure 8** shows that human DCs present proinsulin C19-A3 peptide from GNPs to T cells. Immature DCs were incubated with soluble proinsulin C19-A3 peptide (red bar), with proinsulin C19-A3-coupled GNPs (C19-A3 GNP), at final peptide conc. 10 µg/ml, or with a corresponding dose of GNPs alone (no pep-GNPs), for 4hrs, after which free NPs were washed and DCs matured with LPS for 48hrs. Subsequently, mature DCs were incubated overnight with a proinsulin C19-A3-specific Treg clone that produces IL-10 in response to cognate reaction with DCs. IL-10 in supernatant was measured using Luminex.

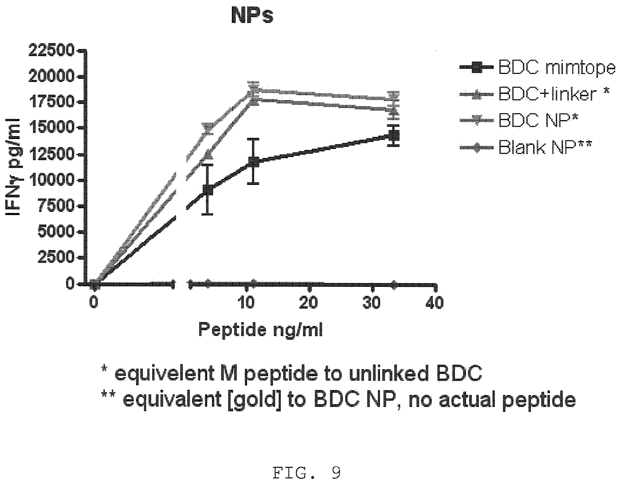

**Figure 9** shows presentation of the BDC2.5 mimotope peptide recognised by BDC-2.5 clonal T cells in mouse spleen cells from BDC-2.5 transgenic mice in vitro. A 3 amino-acid "linker" (AAY) was added to the peptide to allow it to be covalently attached to the GNPs.

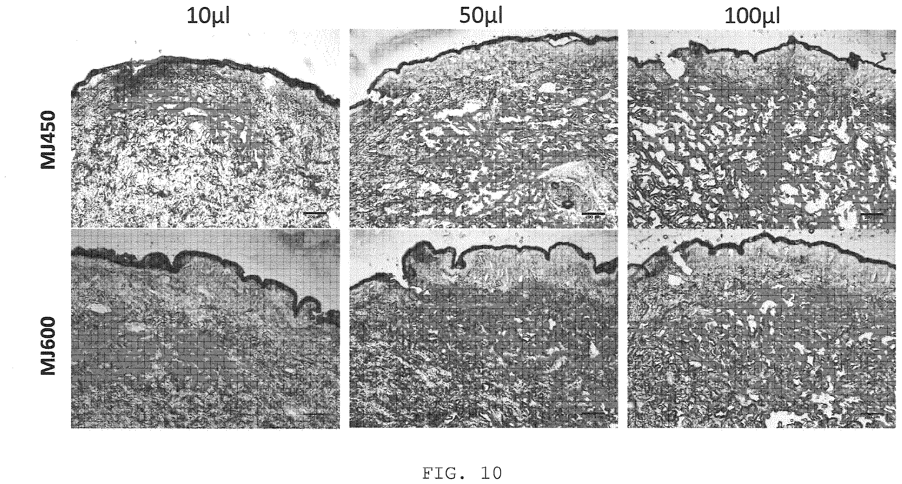

**Figure 10** shows the gross distribution of 30 nm fluorescent latex nanoparticles immediately after injection, using either MJ450 or MJ600 MicronJet microneedles, in fluorescent microscope images of cryosectioned human skin.

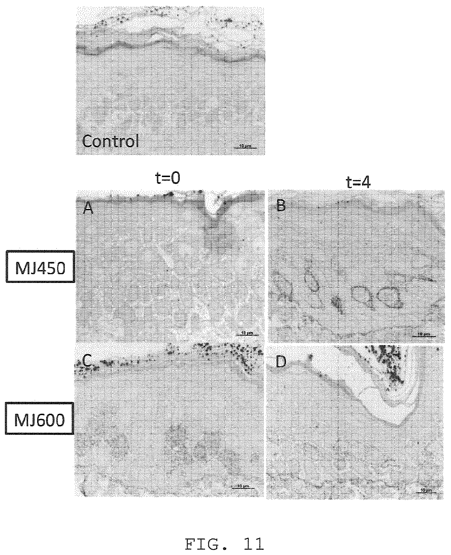

**Figure 11** shows light microscopy images of GNPs (NP23: 90% glutathione and 10% glucose C2) injected into fresh human skin using 450µm (A,B) or 600µm (C,D) MicronJet microneedles. Tissue biopsies were fixed at t=0h and t=4h and analysed for gold particle distribution within the epidermis.

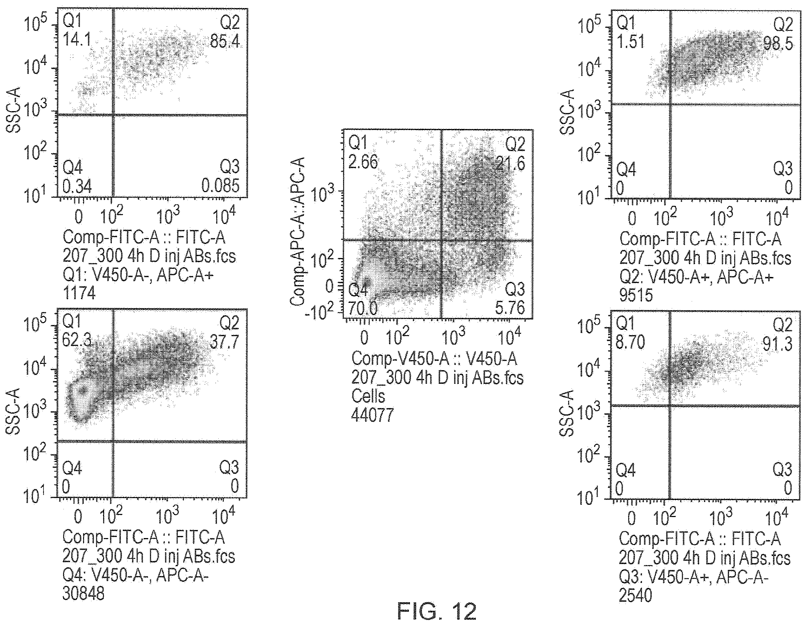

**Figure 12** shows the flow cytometric analysis of dermal cells isolated from skin 4 hours after MicronJet 600 µm microneedle injection of fluorescent 3% peptide C19-A3 GNPs. Total cells were gated for HLADR/CD11c (centre) with each quadrant analysed for FITC. For example the upper right panel represents HLA-DR/CD11c double positive cells (i.e. dermal dendritic cells) and shows that nearly 100% of these cells are positive for FITC.

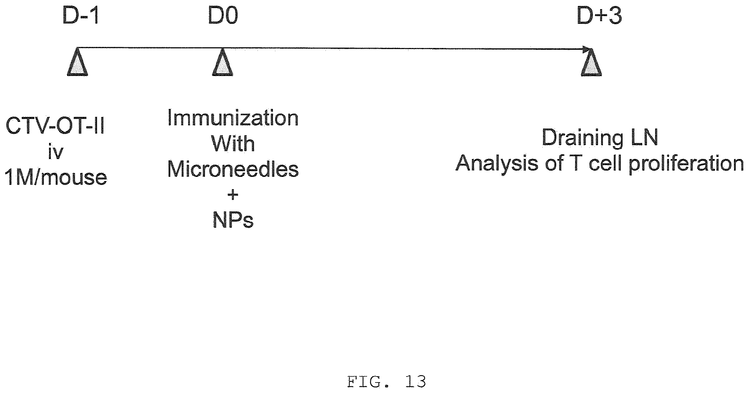

**Figure 13** shows the design of the experimental system used to investigate the transport of GNP-coupled peptides to LNs by DCs.

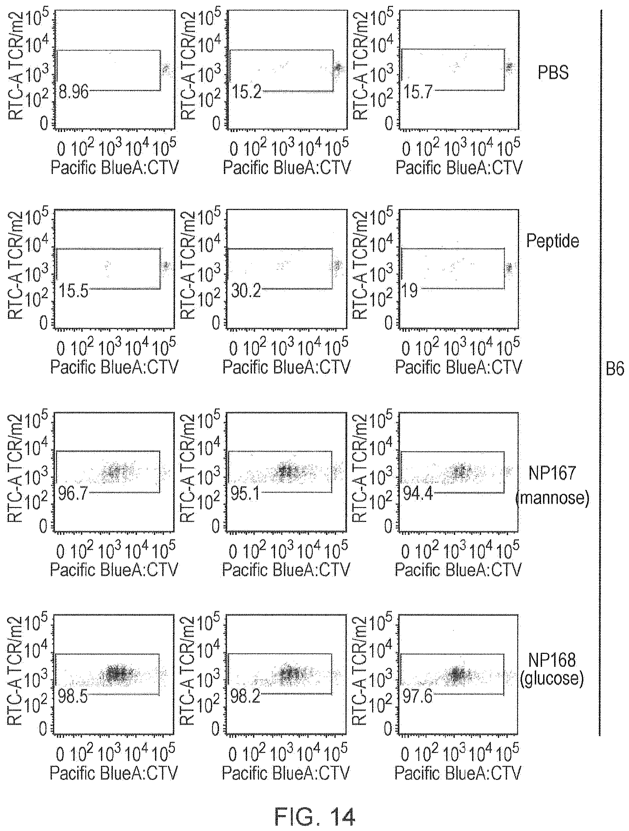

**Figure 14** shows that GNPs are highly efficient at delivering peptide to dendritic cells which subsequently present this peptide to T cells in the draining LN. The small fraction of free peptide that may be found in the GNPs does not account for the proliferation of the T cells. PBS, ovalbumin peptide (323-339) or ovalbumin peptide (323-339) containing GNPs (NP167, NP168) were injected intradermally in C57BL/6 mice 24h after i.v. administration of fluorescent cell trace violet (CTV)-labelled OT-II (ovalbumin peptide (323-339) specific) CD4+ T cells. 3 days later, draining LN cells were isolated and studied by flow cytometry. The presence of OT-II cells in the LN was shown by the appearance of cells of that are CD4⁺ CD45.1⁺Vα2⁺ and high CTV fluorescence (right hand edge of square). Proliferation of OT-II cells was seen as dilution of the CTV fluorescence, bringing the cells progressively at each cell cycle into the blue box. The percentage of cells with the proliferation square is shown. Injection of PBS or peptide alone, results in low levels of OT-II cell accumulation and proliferation, whereas much higher levels are seen with ovalbumin peptide (323-339) bearing GNPs. Each plot represents the results from a different mouse.

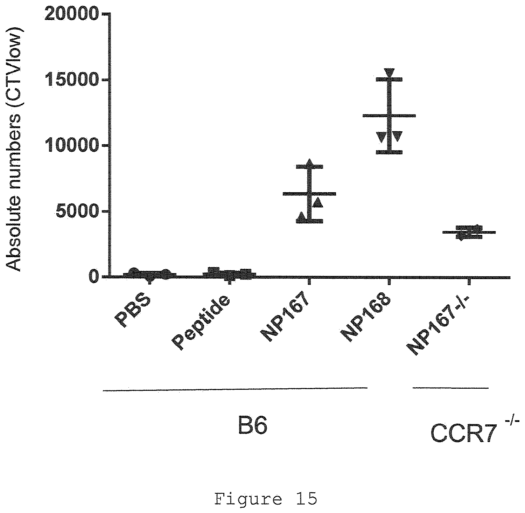

**Figure 15** shows that reduced numbers of fluorescent cells are accumulated in the LN following intradermal injection of ovalbumin (323-339) GNPs in homozygous knockout mice for the chemokine receptor CCR7 (CCR7-/-)that mediates DC migration from skin as compared to wildtype B6 mice. Reduced numbers of fluorescent reported cells accumulated in the LN following intradermal injection of ovalbumin (323-339) GNPs in these mice, suggesting that at least part of the transfer of antigen to the LN is via migratory DCs.

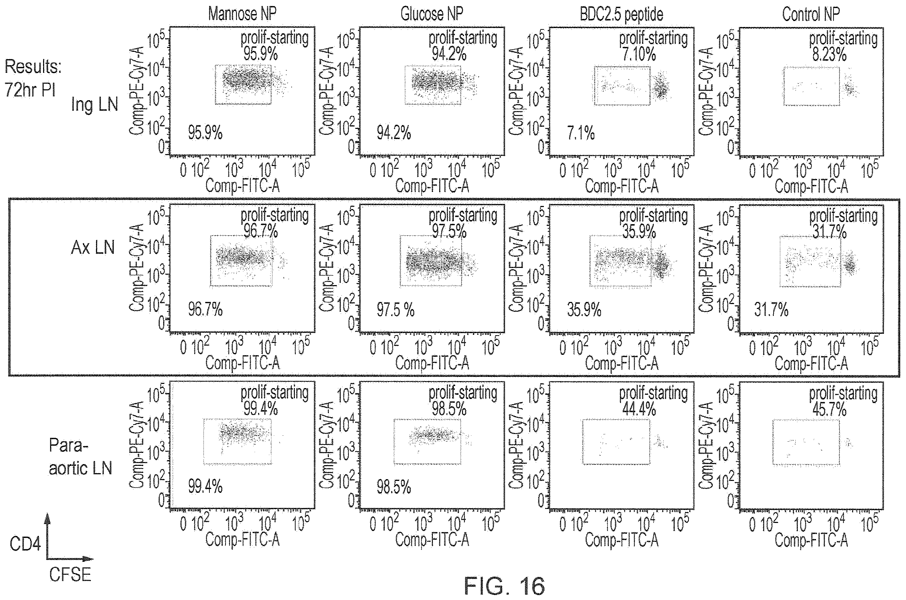

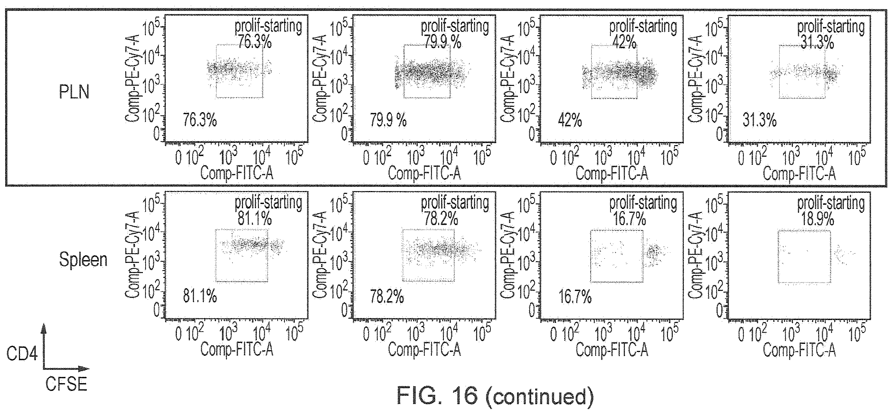

**Figure 16** shows the level of proliferation of indicator fluorescent carboxyfluorescein succinimidyl ester (CFSE) labelled BDC-2.5 CD4⁺ T cells in the draining LN (Ax LN), other LNs including the pancreatic LN (PLN) and spleen, 3 days after injection of BDC-2.5 mimotope peptide coupled GNPs (with mannose or glucose C2), BDC-2.5 mimotope peptide alone, or GNPs with no peptide. Increased proliferation is seen with GNPs coupled to antigen both in the draining and distant LN sites and spleen.

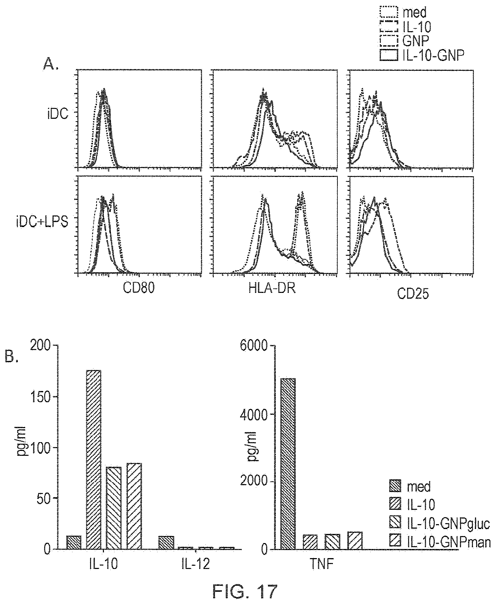

**Figure 17** shows that proinsulin C19-A3 GNPs coupled to IL-10 prevent maturation of DCs and reduce pro-inflammatory cytokine production. (A) Immature human DCs were treated with soluble IL-10 (light blue histogram), proinsulin C19-A3 GNPs (red histogram), or with proinsulin C19-A3 GNPs coupled to IL-10 (dark blue histogram). Upper row shows phenotype of iDCs treated and left alone (upper row) or additionally stimulated with LPS (lower row). (B) Cytokine production by DCs after a treatment with soluble IL-10 (light blue bar), or IL-10-coupled GNPs containing glucose (dark blue bar) or mannose (green bar), and a subsequent stimulation with LPS.

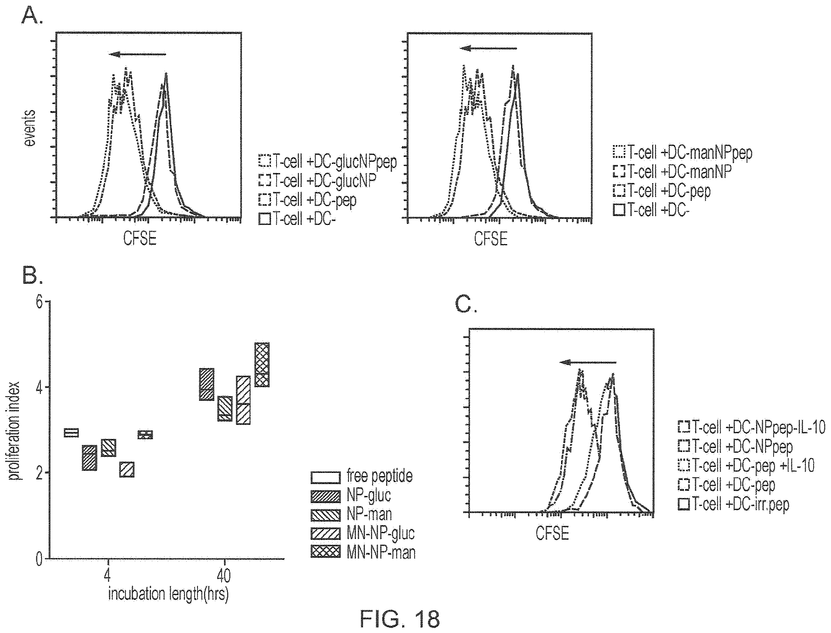

**Figure 18** shows that (A) DCs are able to retrieve peptides delivered by GNP and present them to T cells. iDCs were pre-incubated with GAD(339-352) peptide coupled GNPs for 4 hr and subsequently stimulated with LPS overnight. Thereafter, collected and thoroughly washed DCs were incubated with CFSE-labelled GAD(339-352)-specific CD4⁺ T cells for 72 hrs. If stimulated to proliferate, T cells reduce CFSE staining as depicted by the arrow. (B) GNPs applied through microneedles are taken up, processed and presented to T cells. Proliferation of T cells was compared after stimulation with DCs pulsed with GAD(339-352) peptide alone (grey bar), GAD(339-352) peptide coupled to glucose-GNP (dark blue bars), GAD(339-352) peptide coupled to mannose-GNP (dark red bars), or GAD(339-352) peptide-GNPs applied through microneedles (MN) (hatched bars glucose - blue, mannose - red). GNPs were washed after 4 hr incubation (left) or left with DCs also during the maturation step (right). Proliferation index was calculated by measuring CFSE dilution of proliferating T cells. No reduction in proliferation was seen when peptide/GNPs were provided via microneedles. (C) Combination treatment of DCs with IL-10 and GADpep-GNP reduces activation of T cells. CFSE-labelled T cells were incubated with DCs that have been exposed to: irrelevant peptide (empty histogram), free GAD peptide (grey), GADpep-GNPs (red), soluble GADpep combined with soluble IL-10 (light blue) or GADpep-GNP coupled to IL-10 (dark blue histogram). Proliferation of T cells was evaluated as described in (A) .

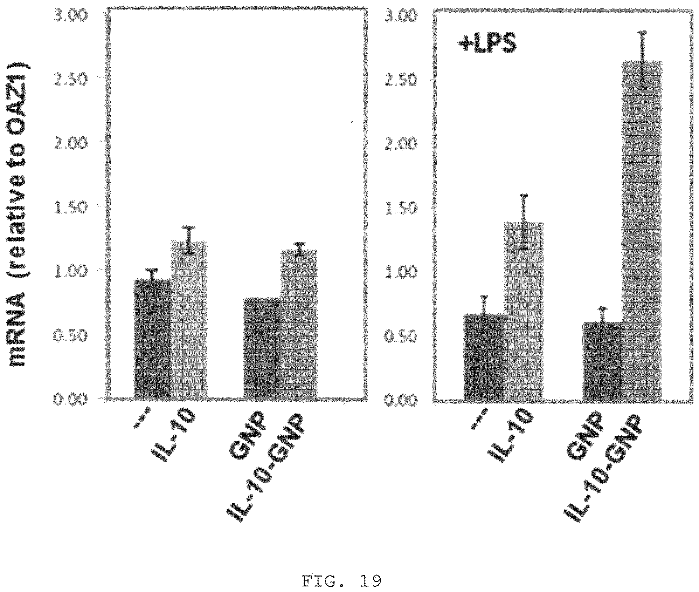

**Figure 19** shows that GNPs coupled to IL-10 induce *de-novo* synthesis of IL-10 mRNA in dendritic cells. Immature huDC were incubated with recombinant IL-10, GNP_{c19-a3} alone or coupled to IL-10 for 4 hours, after which cells were washed and medium refreshed containing GM-CSF alone (800 U/ml) or combined with 100ng/ml LPS. IL-10 added to the cultures (alone or on GNP) was set to 100ng/ml. After 18hrs of culture, cells were washed, mRNA isolated and measured using RT-PCR. Relative expression was calculated using a standard housekeeping gene OAZ1.

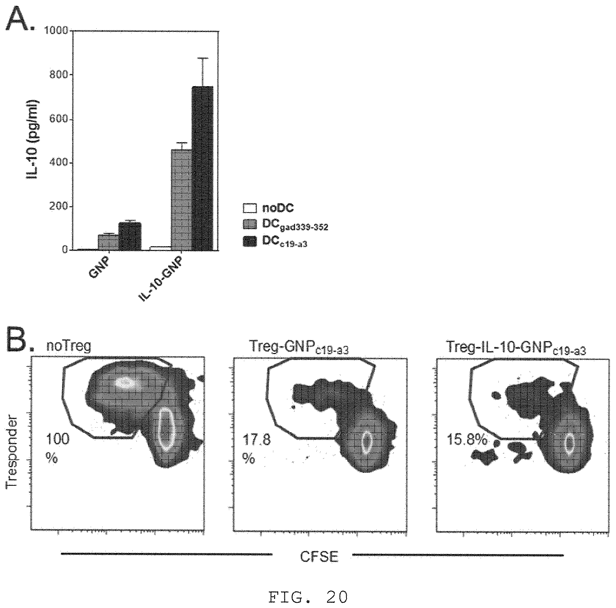

**Figure 20** shows huDCs stimulated with GNP_{c19-a3} induce IL-10 producing inhibitory T cells *in vitro.* Immature huDC were incubated with recombinant GNP_{c19-a3} alone or coupled to IL-10 overnight, after which DCs were washed and used to stimulate autologous naive isolated CD4+ T cells. After a 2-week culture (ref. Unger et al. EJI 2009), cytokine production (**A**) and suppressive capacity (**B**) of remaining T cells were tested. For **A,** Treg lines were stimulated with mature DCs pulsed with either c19-a3 peptide (relevant Ag) or GAD339-352 peptide (irrelevant Ag) and cytokine production after O/N culture was measured. Figure 20A demonstrates antigen-dependent increase in IL-10 production by T cells and a superior capacity to produce IL-10 by T cells stimulated with the IL-10-coupled GNP_{c19-a3}. For **B,** naive CD4+ responder cells isolated form a HLA-mismatched donor were labeled with CFSE and co-incubated with cells as described in A. After a 3-day co-culture, cell proliferation (CFSE-dilution) was determined using Flow-Jo software. Figure 20B shows a strong decrease in proliferation of Tresponder cells when co-incubated with Tregs generated with GNP-treated DCs.

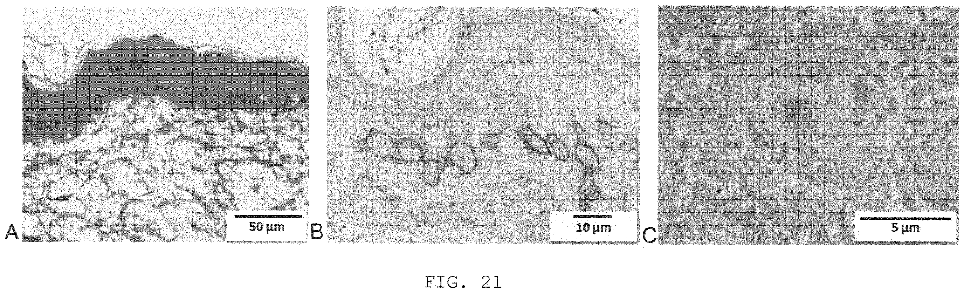

**Figure 21** shows **A, B**) Light microscopy images of 2-4 nm gold nanoparticles injected into fresh human skin using MicronJet microneedles to show "reflux" distribution of particles into the epidermis following intradermal injection. Tissue biopsies were fixed at t=4h and analysed for the distribution of two different types of gold nanoparticles at two different concentrations. **A**) NP 188 containing 3% of C19A3 peptide and 5% glucose were injected at a concentration of 338 µg/ml (with respect to gold). At low magnification these nanoparticles are observed in the dermis, at the junction of the dermis and epidermis and in the epidermis. **B**) NP23: (90%GSH/10%Glc) were injected at a concentration of 20 µg/ml (with respect to gold). At higher magnification, focussing only on the epidermis, these nanoparticles are seen located around and within epidermal cells. **C**) Transmission electron micrograph of a single epidermal cell following injection of gold NPs (NP23: 90%GSH/10%Glc) into fresh human skin using MicronJet microneedles. Nanoparticles are seen within the epidermal cell.

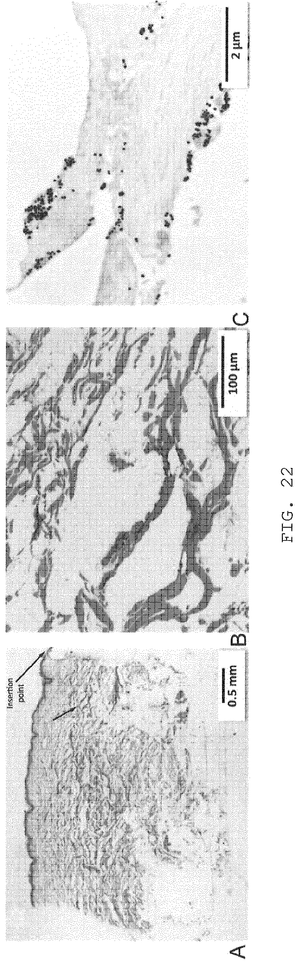

**Figure 22** shows **A, B**) Light microscopy images of 50 nm colloidal gold nanoparticles injected into fresh human skin using MicronJet microneedles to illustrate that these larger particles remain confined to the dermis and do not "reflux" into the epidermis. Tissue biopsies were fixed at t=4h and analysed for gold particle distribution. The same sample of human skin and the same processing conditions and staining protocols were used as in Figure 21A. Both **A)** (low magnification) and **B)** (higher magnification focussing only on the dermis) show that larger colloidal gold nanoparticles are observed in the dermis. No nanoparticles were observed in the epidermis. **C**) Transmission electron micrograph of colloidal gold nanoparticles associated with collagen and elastic fibres in the dermis.

### Detailed description of the invention

In describing the present invention, the following terms will be employed, and are intended to be defined as indicated below.

### Nanoparticles

As used herein, "nanoparticle" refers to a particle having a nanomeric scale, and is not intended to convey any specific shape limitation. In particular, "nanoparticle" encompasses nanospheres, nanotubes, nanoboxes, nanoclusters, nanorods and the like. In certain embodiments the nanoparticles and/or nanoparticle cores contemplated herein have a generally polyhedral or spherical geometry.

Nanoparticles comprising a plurality of carbohydrate-containing ligands have been described in, for example, WO 2002/032404, WO 2004/108165, WO 2005/116226, WO 2006/037979, WO 2007/015105, WO 2007/122388, WO 2005/091704 and such nanoparticles may find use in accordance with the present invention.

As used herein, "corona" refers to a layer or coating, which may partially or completely cover the exposed surface of the nanoparticle core. The corona includes a plurality of ligands which generally include at least one carbohydrate moiety, one surfactant moiety and/or one glutathione moiety. Thus, the corona may be considered to be an organic layer that surrounds or partially surrounds the metallic core. In certain embodiments the corona provides and/or participates in passivating the core of the nanoparticle. Thus, in certain cases the corona may include a sufficiently complete coating layer substantially to stabilise the metal-containing core. However, it is specifically contemplated herein that certain nanoparticles having cores, e.g., that include a metal oxide-containing inner core coated with a noble metal may include a corona that only partially coats the core surface. In certain cases the corona facilitates solubility, such as water solubility, of the nanoparticles of the present invention.

Nanoparticles are small particles, e.g. clusters of metal atoms that can be used as a substrate for immobilising ligands.

Preferably, the nanoparticles have cores having mean diameters between 0.5 and 50 nm, more preferably between 0.5 and 10 nm, more preferably between 0.5 and 5 nm, more preferably between 0.5 and 3 nm and still more preferably between 0.5 and 2.5 nm. When the ligands are considered in addition to the cores, preferably the overall mean diameter of the particles is between 2.0 and 20 nm, more preferably between 3 and 10 nm and most preferably between 4 and 5 nm. The mean diameter can be measured using techniques well known in the art such as transmission electron microscopy.

The core material can be a metal) and may be formed of more than one type of atom. Preferably, the core material is a metal selected from Au, Fe or Cu. Nanoparticle cores may also be formed from alloys including Au/Fe, Au/Cu, Au/Gd, Au/Fe/Cu, Au/Fe/Gd and Au/Fe/Cu/Gd, and may be used in the present invention. Preferred core materials are Au and Fe, with the most preferred material being Au. The cores of the nanoparticles preferably comprise between about 100 and 500 atoms (e.g. gold atoms) to provide core diameters in the nanometre range. Other particularly useful core materials are doped with one or more atoms that are NMR active, allowing the nanoparticles to be detected using NMR, both *in vitro* and *in vivo.* Examples of NMR active atoms include Mn⁺², Gd⁺³, Eu⁺², Cu⁺², V⁺², Co⁺², Ni⁺², Fe⁺², Fe⁺³ and lanthanides⁺³, or the quantum dots described elsewhere in this application.

In some embodiments, the nanoparticle or its ligand comprises a detectable label. The label may be an element of the core of the nanoparticle or the ligand. The label may be detectable because of an intrinsic property of that element of the nanoparticle or by being linked, conjugated or associated with a further moiety that is detectable. Preferred examples of labels include a label which is a fluorescent group, a radionuclide, a magnetic label or a dye. Fluorescent groups include fluorescein, rhodamine or tetramethyl rhodamine, Texas-Red, Cy3, Cy5, etc., and may be detected by excitation of the fluorescent label and detection of the emitted light using Raman scattering spectroscopy (Y.C. Cao, R. Jin, C. A. Mirkin, Science 2002, 297: 1536-1539).

In some embodiments, the nanoparticles may comprise a radionuclide for use in detecting the nanoparticle using the radioactivity emitted by the radionuclide, e.g. by using PET, SPECT, or for therapy, i.e. for killing target cells. Examples of radionuclides commonly used in the art that could be readily adapted for use in the present invention include ^{99m}Tc, which exists in a variety of oxidation states although the most stable is TcO⁴⁻; ³²P or ³³P; ⁵⁷Co; ⁵⁹Fe; ⁶⁷CU which is often used as Cu²⁺ salts; ⁶⁷Ga which is commonly used a Ga³⁺ salt, e.g. gallium citrate; ⁶⁸Ge; ⁸²Sr; ⁹⁹Mo; ¹⁰³Pd; ¹¹¹In which is generally used as In³⁺ salts; ¹²⁵I or ¹³¹I which is generally used as sodium iodide; ¹³⁷CS; ¹⁵³Gd; ¹⁵³Sm; ¹⁵⁸Au; ¹⁸⁶Re; ²⁰¹Tl generally used as a Tl⁺ salt such as thallium chloride; ³⁹Y³⁺; ⁷¹Lu³⁺; and ²⁴Cr²⁺. The general use of radionuclides as labels and tracers is well known in the art and could readily be adapted by the skilled person for use in the aspects of the present invention. The radionuclides may be employed most easily by doping the cores of the nanoparticles or including them as labels present as part of ligands immobilised on the nanoparticles.

### Autoantigen

As used herein, an "autoantigen" (also known as a self-antigen) is an antigen that despite being a normal tissue constituent is the target of a humoral or cell-mediated immune response, as in autoimmune disease. An autoantigen peptide is a peptide, polypeptide or fragment thereof, that is or acts as an autoantigen. An autoantigen peptide may in some cases further comprise a non-peptide portion or modification, for example, an autoantigen peptide may be a glycosylated peptide. In particular, the autoantigen may be a hybrid peptide or polypeptide formed by combining (e.g. as a fusion protein) two or more different autoantigen peptides or epitope-containing parts thereof. For example, an autoantigen peptide may comprise proinsulin or a fragment of proinsulin combined with or fused to another autoantigen such as another type 1 diabetes-related autoantigen peptide. Equally, the autoantigen peptide may comprise two or more repeats of the same autoantigen peptide.

In particular cases herein, the autoantigen peptide may be a peptide, polypeptide or fragment thereof that is the target of an immune response in a disease selected from the group consisting of:
type 1 diabetes (e.g. insulin or proinsulin or fragment thereof (e.g. proinsulin C19-A3), glutamic acid decarboxylase (GAD), IA2, islet cell antigens, chromogranin A (e.g. chromograninA 342-355 or synthetic peptide mimicking chromograninA 342-355 peptide, the BDC2.5 mimotope), zinc transporter 8(ZnT8/Slc30A8)) a hybrid peptide having the amino acid sequence DLQTLALWSRMD (SEQ ID NO: 12);
multiple sclerosis (MS) (e.g. myelin basic protein (MBP));
primary biliary cirrhosis (PBC) (e.g. pyruvate dehydrogenase complex (PDC-E2);
myasthenia gravis (e.g. nicotinic acetylcholine receptor (nAChR);
rheumatoid arthritis (RA) (e.g. collagen type II); and
autoimmune thyroid disease (e.g. thyroid peroxidase, thyroglobulin and TSH receptor).

In particular cases herein, the autoantigen peptide may be selected from the group consisting of:
Proinsulin peptide C19-A3 having the amino acid sequence GSLQPLALEGSLQKRGIV (SEQ ID NO: 1);
Glutamic acid decarboxylase (GAD) (339-352) having the amino acid sequence TVYGAFDPLLAVAD (SEQ ID NO: 2);
BDC-2.5 mimotope peptide having the amino acid sequence YVRPLWVRME (SEQ ID NO: 3);
ChromograninA (342-355) peptide - WE-14 having the amino acid sequence WSRMDQLAKELTAE (SEQ ID NO: 4) (murine form) or WSKMDQLAKELTAE (human form) (SEQ ID NO: 6);
Insulin B (9-23) peptide having the amino acid sequence SHLVEALYLVSGERG (SEQ ID NO: 5);
human proinsulin peptide having the amino acid sequence or
a variant thereof differing by addition, substitution or deletion of not more than 5, 4, 3, 2, or not more than 1 amino acid; and
a hybrid peptide having the amino acid sequence DLQTLALWSRMD (SEQ ID NO: 12),
or a hybrid peptide comprising two or more of said peptides linked or fused together (whether two or more of the same peptide or two or more different peptides).

### Anti-inflammatory cytokine

As used herein, the term "anti-inflammatory cytokine" is intended to mean an immunoregulatory molecule that controls or down-regulates the proinflammatory response. In particular, the anti-inflammatory cytokine may be selected from the group consisting of: (IL)-1 receptor antagonist, IL-4, IL-5, IL-10, IL-11, IL-13, TGFbeta1 and TGFbeta2. In particular cases the anti-inflammatory cytokine is interleukin 10 (IL-10), preferably human IL-10. IL-10 has the UniProt accession number P22301 (Last modified: August 1, 1991 - version 1; Checksum: 6825E9FA4337CDE4).

### Carbohydrate

As used herein, the term "carbohydrate" is intended to include compounds of the general formula Cₙ(H₂O)ₘ wherein n = m and n is greater than 3. Also included within the definition of carbohydrate are carbohydrate analogues / mimetics that are not included in the general formula Cₙ(H₂O)ₘ. The carbohydrate analogues include pseudo-sugars (carba-sugars), amino-sugars, imino-sugars and inositols. Amino sugars include polyhydroxylated piperidines, pyrrolidines, pyrrolizidines and indolizidines. In particular cases, the carbohydrate is a monosaccharide, such as glucose, mannose, galactose, glucosamine or N-acetylglucosamine. As used herein, the term "carbohydrate ligand" includes a ligand having a carbohydrate moiety attached to a linker, which may in turn be covalently attached to the core of the nanoparticle. In particular, a carbohydrate ligand may be a glycoside (such as a glycoside of glucose or mannose, e.g., a glucopyranoside). The carbohydrate ligand may be covalently linked to the core via a linker selected from sulphur-containing linkers, amino-containing linkers and phosphate-containing linkers. Combinations of linkers off of the core may also be used. The linker may in some cases include a linear chain of at least two atoms (e.g. between 2 and 30 atoms), including for example an alkyl or glycol chain of at least two carbon atoms (e.g. 2-12, 2-8 or 2-5).

### Glutathione

Glutathione (IUPAC name (2S)-2-amino-4-{[(1R)-1-[(carboxymethyl)carbamoyl]-2-sulfanylethyl]carbamoyl}butanoic acid) is a tripeptide with a gamma peptide linkage between the carboxyl group of the glutamate side-chain and the amine group of cysteine. Glutathione exists in both reduced (GSH) and oxidized (GSSG) states. In the reduced state, the thiol group of cysteine is able to donate a reducing equivalent to other unstable molecules, such as reactive oxygen species. In donating an electron, glutathione itself becomes reactive, but readily reacts with another reactive glutathione to form glutathione disulfide (GSSG).

### Microneedle

The microneedle system as used herein is preferably MicronJet intradermal needles available from Nanopass Technologies Ltd., Nes Ziona, Israel. See, e.g., Van Damme P, et al. Vaccine. 2009 Jan 14;27(3):454-9, and Levin Y, et al. Vaccine. 2014. Clinical evaluation of a novel microneedle device for intradermal delivery of an influenza vaccine: Are all delivery methods the same?. Vaccine, 32(34), 4249-4252.

### Administration and treatment

The nanoparticles and compositions of the invention may be administered to patients by any number of different routes, including intradermal microneedle injection, enteral or parenteral routes. Parenteral administration includes administration by the following routes: intravenous, cutaneous or subcutaneous, nasal, intramuscular, intraocular, transepithelial, intraperitoneal and topical (including dermal, ocular, rectal, nasal, inhalation and aerosol), and rectal systemic routes.

Administration be performed e.g. by injection, especially microneedle injection.

The nanoparticles of the invention may be formulated as pharmaceutical compositions that may be in the forms of solid or liquid compositions. Such compositions will generally comprise a carrier of some sort, for example a solid carrier or a liquid carrier such as water, petroleum, animal or vegetable oils, mineral oil or synthetic oil. Physiological saline solution, or glycols such as ethylene glycol, propylene glycol or polyethylene glycol may be included. Such compositions and preparations generally contain at least 0.1 wt% of the compound.

For intradermal, intravenous, cutaneous or subcutaneous injection, or injection at the site of affliction, the active ingredient will generally be in the form of a parenterally acceptable aqueous solution or liquid which is pyrogen-free and has suitable pH, isotonicity and stability. Those of relevant skill in the art are well able to prepare suitable solutions using, for example, solutions of the compounds or a derivative thereof, e.g. in physiological saline, a dispersion prepared with glycerol, liquid polyethylene glycol or oils.

In addition to one or more of the compounds, optionally in combination with other active ingredient, the compositions can comprise one or more of a pharmaceutically acceptable excipient, carrier, buffer, stabiliser, isotonicising agent, preservative or anti-oxidant or other materials well known to those skilled in the art. Such materials should be non-toxic and should not interfere with the efficacy of the active ingredient. The precise nature of the carrier or other material may depend on the route of administration, e.g., intramuscular injection.

Preferably, the pharmaceutically compositions are given to an individual in a prophylactically effective amount or a therapeutically effective amount (as the case may be, although prophylaxis may be considered therapy), this being sufficient to show benefit to the individual. Typically, this will be to cause a therapeutically useful activity providing benefit to the individual. The actual amount of the compounds administered, and rate and time-course of administration, will depend on the nature and severity of the condition being treated. Prescription of treatment, e.g. decisions on dosage etc., is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Handbook of Pharmaceutical Additives, 2nd Edition (eds. M. Ash and I. Ash), 2001 (Synapse Information Resources, Inc., Endicott, New York, USA); Remington's Pharmaceutical Sciences, 20th Edition, 2000, pub. Lippincott, Williams & Wilkins; and Handbook of Pharmaceutical Excipients, 2nd edition, 1994. By way of example, the compositions are preferably administered to patients in dosages of between about 0.01 and 100 microgrammes of active compound per subject, and more preferably between about 0.5 and 100 microgrammes.

The following is presented by way of example and is not to be construed as a limitation to the scope of the claims.

### Examples

### Example 1 - Synthesis of gold nanoparticles (GNPs) with covalently autoantigen peptide payloads

### 1.1 WE-14 Peptide

### Summary

The aims of this experiment were to generate 5% mannose C2 and 5% glucose C2 gold nanoparticles (GNPs) conjugated to WE14 murine peptide (0.5%, 1%, 2.5%) to determine incorporation efficiency of WE14 peptide using the 10% methanol preparation method.

Methods. 5% mannose C2 and 5% glucose C2 GNPs were covalently conjugated to WE14 from chromogranin-A (342-355) murine peptide [-S(CH₂)₂- CONH-AAYWSRMDQLAKELTAE]₂ (SEQ ID NO: 8) (WE14 peptide sequence underlined) (0.5%, 1%, and 2.5%) and glutathione (94.5%, 94%, 92.5%) using the water-based preparation method. The AAY linker at the N-terminus of the WE14 peptide sequence was employed for WE14 peptide attachment (see WO2013/034726). Particle sizes were determined using dynamic light scattering (DLS) and peptide incorporation into the NP was determined using HPLC, following the addition of potassium cyanide solution (KCN) to disintegrate the NP.

Results. WE14 peptide was found to be incorporated with 91-100% efficiency into both 5% glucose C2 and 5% mannose C2 GNPs. The numbers of peptides bound per GNP were 0.85 and 1.4 for NP133 and NP136, respectively, containing 0.5% WE14 peptide, and increased with higher WE14 peptide concentration to 2.8 for both NP134 and NP137 containing 1% WE14 peptide and 8 and 7.6 for NP135 and NP138, respectively, containing 2.5% WE14 peptide. The proportion of GNPs conjugated to WE14 peptide was 99-100% for all preparations.

Conclusions. WE14 peptide was incorporated with high efficiency into 5% glucose C2 and 5% mannose C2 GNPs using the 10% methanol preparation method. WE14 peptide concentrations of 0.5% or 1% were found to be optimal for conjugation, with ∼1-1.4 peptides/GNP and 2.8 peptides bound GNP, respectively.

### Methods

WE14 peptide from chromogranin-A (342-355) murine peptide [-S(CH₂)₂-CONH-AAYWSRMDQLAKELTAE]₂ (SEQ ID NO: 8) (AmbioPharm, Inc., SC, USA) was synthesised with a thiol proprionic acid linker in an amide linkage at the N terminal. WE14 peptide (0.5%, 1% or 2.5%) was dissolved in water (2 mg/ml). For all GNP preparations, 10 pmol gold was mixed with 5% glucose C2 or 5% mannose C2, glutathione and WE14 peptide ligands (in a 3 fold excess to gold) (Table 1) and the reaction mixture made up to 2 ml with 10% methanol.

**Table 1. Preparation of WE14-conjugated GNPs**

| **ID** | **WE14** | | | **5% Mannose** | | **5% Glucose** | | **Gold** | |
|---|---|---|---|---|---|---|---|---|---|
| | **%** | **µg** | **pmol** | **µg** | **pmol** | **µg** | **pmol** | **µl** | **µmol** |
| **NP133** | 0.5 | 621.1 | 0.15 | 360.41 | 1.5 | - | - | 16.3 | 10 |
| **NP134** | 1 | 1242.2 | 0.3 | 360.41 | 1.5 | - | - | 16.3 | 10 |
| **NP135** | 2.5 | 3105.5 | 0.75 | 360.41 | 1.5 | - | - | 16.3 | 10 |
| **NP136** | 0.5 | 621.1 | 0.15 | - | - | 363 | 1.5 | 16.3 | 10 |
| **NP137** | 1 | 1242.2 | 0.3 | - | - | 363 | 1.5 | 16.3 | 10 |
| **NP138** | 2.5 | 3105.5 | 0.75 | - | - | 363 | 1.5 | 16.3 | 10 |

Sodium borohydride (200 pmol; 7.6 mg in 200 µl water) was then quickly added to the reaction tubes in one go with continuous vortexing for 1 min. The reaction tubes were then incubated for 1 h at room temperature with constant mixing. After 1 h, the solutions were transferred to Amicon centrifugal filter tubes (Millipore Ltd, 10 K membrane molecular weight cut-off) and centrifuged at 5000 rpm for 6 min to remove unbound WE14 peptide. The supernatant was removed and stored at 4°C. The concentrated NPs on top of the membrane were then washed 4 times by adding 2 ml of water each time and centrifuging at 5000 rpm for 6 min each to ensure removal of unbound WE14 peptide. The volume of the concentrated NPs was made up to 1 ml with water and the NP solutions stored at 4°C until analysed. A gold assay was performed to determine gold content of WE14 peptide conjugated glucose or mannose C2 GNPs (Table 2). DLS was performed to determine NP size (in 35% PBS solution) of WE14 peptide conjugated GNPs (Table 3).

**Table 2: Gold content of 5% glucose C2 or mannose C2 GNPs, conjugated to either 1% or 2.5% WE14 peptide**

| **NP** | **% BDC2.5** | **Gold content (mg/ml)** | **SD** |
|---|---|---|---|
| **NP133** | 0.5 | 1.517 | 0.059 |
| **NP134** | 1 | 1.792 | 0.021 |
| **NP135** | 2.5 | 1.796 | 0.064 |
| **NP136** | 0.5 | 1. 628 | 0.034 |
| **NP137** | 1 | 1. 658 | 0.061 |
| **NP138** | 2.5 | 1.880 | 0.020 |

**Table 3: DLS size results for 5% glucose or mannose C2 GNPs, conjugated with 1% or 2.5% WE14 peptide**

| | **% BDC2.5** | **Mean size (nm)** | **SD** |
|---|---|---|---|
| **NP133** | 0.5 | 6.63 | 0.42 |
| **NP134** | 1 | 3.29 | 0.95 |
| **NP135** | 2.5 | 7.23 | 0.52 |
| **NP136** | 0.5 | 6.94 | 3.35 |
| **NP137** | 1 | 7.76 | 0.43 |
| **NP138** | 2.5 | 7.80 | 1.04 |

### HPLC methods and results

WE14 standard (2 µg; 20 µl) was injected for HPLC analysis and served as a reference standard. To determine the amount of WE14 peptide incorporated into the GNPs, 40 µl of 100 mM KCN in 10 mM KOH was added to 5 µl NP, of which 20 µl was injected for HPLC analysis. HPLC chromatograms (not shown) were acquired at 212 nm and 400 nm. Aliquots of each GNP (10 µl) were diluted with TFA (20 µl), of which 20 µl was injected onto HPLC to determine the proportion of GNP with bound peptide and to determine whether any free peptide remained in these NP solutions, following centrifugation using the Amicon centrifugal filter tubes.

**Table 4: Summary of HPLC Results**

| **ID** | **% WE14** | **% WE14 incorporation** | **% NPs with bound WE14** | **Number of peptides/NP** |
|---|---|---|---|---|
| **NP133** | 0.5 | 56 | 100 | 0.85 |
| **NP134** | 1 | 94 | 99 | 2.8 |
| **NP135** | 2.5 | 107 | 100 | 8 |
| **NP136** | 0.5 | 91.4 | 98.5 | 1.4 |
| **NP137** | 1 | 94 | 99 | 2.8 |
| **NP138** | 12.5 | 101 | 99.4 | 7.6 |

### 1.2 BDC-2.5 Mimotope Peptide

### Summary

The aims of this experiment were to generate 5% mannose C2 and 5% glucose C2 GNPs conjugated to BDC-2.5 mimotope murine peptide (1%, 2.5%) using the 75% methanol-based preparation method and to determine the incorporation efficiency of BDC2.5 peptide using HPLC. Methods. BDC2.5 mimotope murine peptide ([-S(CH₂)₂- CONH-AAYYVRPLWVRME]₂₎ (SEQ ID NO: 9) (shown underlined with the AAY peptide linker at the N-terminus) was dissolved in DMSO (50 mg/ml). Mannose C2 and glucose C2 (5%) GNPs were covalently conjugated to BDC2.5 mimotope peptide (1%, 2.5%) and glutathione (94%, 92.5%) using the 75% methanol-based preparation method. Particle sizes were determined using dynamic light scattering (DLS) and peptide incorporation into the GNP was determined using HPLC, following the addition of potassium cyanide solution (KCN) to disintegrate the NP. Results. BDC2.5 mimotope murine peptide (1% or 2.5%) was found to be incorporated with 28-38% efficiency into 5% glucose C2 or 5% mannose C2 GNPs, with 95-99% of GNPs bound with BDC2.5 mimotope peptide. The number of bound peptides/GNP was 0.9 for mannose C2 (NP125) and glucose C2 (NP127) GNPs, containing 1% BDC2.5 mimotope peptide, and increased to 2.8 for mannose C2 GNPs (NP126) and 2.14 for glucose C2 GNPs (NP128) containing 2.5% BDC2.5 mimotope peptide.

Conclusions. BDC2.5 mimotope murine peptide was incorporated with 28-38% efficiency into 5% glucose C2 and 5% mannose C2 GNPs using the 75% methanol-based preparation method. GNPs conjugated with 1% BDC2.5 mimotope peptide had ∼1 peptide bound per NP while GNPs conjugated with 2.5% BDC2.5 mimotope peptide had ∼2 - 2.8 peptides bound per NP.

### Methods

BDC-2.5 mimotope murine peptide ([-S(CH₂)₂- CONH-AAYYVRPLWVRME]₂₎ (SEQ ID NO: 9) (AmbioPharm, Inc., SC, USA) was synthesised with a thiol proprionic acid linker in an amide linkage at the N terminal. For conjugation to GNPs, BDC-2.5 peptide was first dissolved in DMSO (50 mg/ml). 5% mannose C2 or glucose C2 GNPs conjugated to BDC-2.5 peptide (1% or 2.5%) were then prepared using the 75% methanol-based preparation method. For all reactions, GNPs were prepared by mixing 10 pmol gold with a 3 fold excess of glucose C2 or mannose C2, glutathione and BDC-2.5 peptide ligands (in a 3 fold excess to gold) (Table 5) and the reaction mixture made up to 2 ml with 75% methanol.

**Table 5. Preparation of BDC-2.5 conjugated GNPs**

| **ID** | **BDC-2.5** | | | **5% Mannose C2** | | **5% Glucose C2** | | **Gold** | |
|---|---|---|---|---|---|---|---|---|---|
| | **%** | **µg** | **µmol** | **µg** | **µmol** | **µg** | **µmol** | **µl** | **µmol** |
| **NP125** | 1 | 327 | 0.3 | 360.41 | 1.5 | - | - | 16.3 | 10 |
| **NP126** | 2.5 | 991 | 0.75 | 360.41 | 1.5 | - | - | 16.3 | 10 |
| **NP127** | 1 | 309.2 | 0.3 | - | - | 363 | 1.5 | 16.3 | 10 |
| **NP128** | 2.5 | 744 | 0.75 | - | - | 363 | 1.5 | 16.3 | 10 |

Sodium borohydride (200 pmol; 7.6 mg in 200 µl water) was then quickly added to the reaction tubes in one go with continuous vortexing for 1 min. The reaction tubes were then incubated for 1 h at room temperature with constant mixing. After 1 h, the solutions were transferred to Amicon centrifugal filter tubes (Millipore Ltd, 10 K membrane molecular weight cut-off) and centrifuged at 5000 rpm for 6 min to remove unbound BDC-2.5 mimotope peptide. The supernatant was removed and stored at 4°C. The concentrated NPs on top of the membrane were then washed 4 times with water by adding 2 ml of water each time and centrifuging at 5000 rpm for 6 min each. The supernatant (8 ml) was removed and stored at 4°C. Water (1 ml) was added to the concentrated NPs and the NP solutions stored at 4°C until analysed. A gold assay was performed to determine gold content of BDC-2.5 mimotope peptide conjugated to the glucose or mannose C2 GNPs (Table 6). DLS was performed to determine NP size (in 35% PBS solution) of BDC-2.5 mimotope peptide conjugated GNPs (Table 7).

**Table 6: Gold content of 5% glucose C2 or 5% mannose C2 GNPs, conjugated to either 1% or 2.5% BDC-2.5 peptide**

| **ID** | **BDC2.5 %** | **Gold (mg/ml)** | **SD** |
|---|---|---|---|
| **NP125** | 1 | 1.755 | 0.042 |
| **NP126** | 2.5 | 1.907 | 0.009 |
| **NP127** | 1 | 1.729 | 0.035 |
| **NP128** | 2.5 | 1.752 | 0.006 |

**Table 7: DLS size results for 5% glucose C2 and 5% mannose C2 GNPs, conjugated with 1% or 2.5% BDC-2.5 peptide**

| | **% BDC-2.5** | **Mean size (nm)** | **SD** |
|---|---|---|---|
| **NP125** | 1 | 3.31 | 0.46 |
| **NP126** | 2.5 | 5.30 | 1.47 |
| **NP127** | 1 | 3.75 | 0.25 |
| **NP128** | 2.5 | 4.78 | 0.35 |

### HPLC methods and results

BDC-2.5 mimotope standard (2 µg; 20 µl) was injected for HPLC analysis and served as a reference standard. To determine the amount of BDC-2.5 mimotope peptide incorporated into GNPs, 40 µl of 100 mM KCN in 10 mM KOH was added to 5 µl GNP, of which 20 µl was injected for HPLC analysis. HPLC chromatograms were acquired at 212 nm and 400 nm. Aliquots of GNPs (10 µl diluted with 20 µl TFA, of which 20 µl was injected onto HPLC) were also analysed on HPLC to determine whether any free peptide remained in the GNP solutions, following centrifugation using the Amicon centrifugal filter tubes.

**Table 8: HPLC results summary**

| **ID** | **% BDC-2.5** | **% BDC-2.5 incorporation** | **% NPs with bound BDC-2.5** | **Number of peptides/NP** |
|---|---|---|---|---|
| **NP125** | 1 | 31 | 97 | 0.94 |
| **NP126** | 2.5 | 38 | 99 | 2.85 |
| **NP127** | 1 | 30 | 95 | 0.9 |
| **NP128** | 2.5 | 28.5 | 98 | 2.14 |

### 1.3 Insulin B (9-23) Peptide

### Summary

The aims of this experiment were to generate 5% mannose C2 and 5% glucose C2 GNPs conjugated to murine insulin B (9-23) peptide (0.5%, 1%, 2%) and to determine the incorporation efficiency of insulin B (9-23) peptide using the water-based preparation method.

Methods. Insulin B (9-23) ([-S(CH₂)₂- CONH-AAYSHLVEALYLVSGERG]₂₎ (SEQ ID NO: 10) (Insulin B(9-23) sequence shown underlined; AAY was added as linker to N-terminus) was dissolved in DMSO (10 mg/ml). 5% mannose C2 and 5% glucose C2 GNPs were covalently conjugated to insulin B (9-23) peptide (0.5% 1%, 2%) and glutathione (94.5%, 94%, 93%) using the water-based preparation method. Particle sizes were determined using dynamic light scattering (DLS) and peptide incorporation into the NP was determined using HPLC, following the addition of potassium cyanide solution (KCN) to disintegrate the NP.

Results. Insulin B (9-23) peptide (1% or 2%) was incorporated with higher efficiency (98%) into 5% mannose C2 GNPs (NP151, NP152) than 5% glucose C2 (45% for NP153 and 55% for NP154, respectively). Mannose GNPs prepared with 1% or 2% insulin B (9-23) peptide had 3 peptides/GNP (NP151) and 6 peptides/GNP (NP153), respectively, while glucose C2 GNPs prepared with 1% or 2% peptide had 1.4 peptides/GNP (NP152) and 3.3 peptides/GNP (NP154), respectively. The proportion of these GNPs conjugated to insulin B (9-23) peptide was 91-98%. However, when a lower concentration of 0.5% insulin B (9-23) peptide was used, incorporation efficiency was reduced to 32% and 61%, with 0.5 and 1 peptide bound per GNP for mannose and glucose C2 GNPs, respectively.

Conclusions. Insulin B (9-23) peptide (1%, 2%) was incorporated with high efficiency into 5% glucose C2 and 5% mannose C2 GNPs using the water-based preparation method. Insulin B (9-23) peptide concentration of 1% was optimal, with ∼3 peptides/GNP for mannose C2 GNPs and 1.4 peptides/GNP for glucose C2 GNPs.

### Methods

Insulin B (9-23) ([-S(CH₂)₂- CONH-AAYSHLVEALYLVSGERG]₂₎ (SEQ ID NO: 10) was dissolved in DMSO (10 mg/ml). 5% mannose C2 or glucose C2 GNPs were then conjugated to insulin B (9-23) peptide (1% or 2%) using a water-based preparation method. For all reactions, GNPs were prepared by mixing 10 pmol gold with a 3-fold excess of glucose C2 or mannose C2 and glutathione ligands. Sodium borohydride (200 pmol; 7.6 mg in 200 µl water) was then quickly added to the reaction tubes in one go with continuous vortexing for 1 min. The reaction tubes were then incubated for 1 h at room temperature with constant mixing. After 1 h, the solutions were transferred to Amicon centrifugal filter tubes (Millipore Ltd, 10 K membrane molecular weight cut-off) and centrifuged at 5000 rpm for 20 min to remove unbound peptide. The supernatant was removed and stored at 4°C. The concentrated NPs on top of the membrane were then washed 4 times with water by adding 2 ml of water each time and centrifuging at 5000 rpm for 10 min, 2x 6 min and 10 min, respectively. Water (1 ml) was added to the concentrated NPs and the NP solutions stored at 4°C until analysed. A gold assay was performed to determine gold content of insulin B (9-23) peptide conjugated to the glucose or mannose C2 GNPs (Table 9). DLS was performed to determine NP size (in 35% PBS solution) of insulin B (9-23) peptide conjugated GNPs (Table 10).

**Table 9: Gold content of 5% glucose C2 or mannose C2 GNPs, conjugated to either 0.5%, 1% or 2.5% insulin B peptide**

| **NP** | **% insulin B** | **% mannose** | **% glucose** | **Gold content (mg/ml)** | **SD** |
|---|---|---|---|---|---|
| **NP151** | 1 | 5 | - | 1.41 | 0.02 |
| **NP152** | 2 | 5 | - | 1.48 | 0.01 |
| **NP153** | 1 | - | 5 | 1.60 | 0.01 |
| **NP154** | 2 | - | 5 | 1.74 | 0.02 |
| **NP157** | 0.5 | 5 | - | 1.72 | 0.01 |
| **NP158** | 0.5 | - | 5 | 1.72 | 0.21 |

**Table 10: DLS size results for 5% glucose or mannose C2 GNPs, conjugated with 0.5%, 1% or 2.5% insulin B (9-23) peptide**

| | **% insulin B** | **% mannose** | **% glucose** | **Mean size (nm)** | **SD** |
|---|---|---|---|---|---|
| **NP151** | 1 | 5 | - | 5.81 | 0.02 |
| **NP152** | 2 | 5 | - | 4.71 | 2.17 |
| **NP153** | 1 | - | 5 | 51.1 | 5.7 |
| **NP154** | 2 | - | 5 | 37.6 | 4.4 |
| **NP157** | 0.5 | 5 | - | 7.64 | 0.54 |
| **NP158** | 0.5 | - | 5 | 8.69 | 0.76 |

### HPLC methods and results

Insulin B (9-23) standard (2.5 µg) was injected for HPLC analysis and served as a reference standard. To determine the amount of peptide incorporated into GNPs, 40 µl of 100 mM KCN in 10 mM KOH was added to 10 µl GNP, of which 20 µl was injected for HPLC analysis. HPLC chromatograms were acquired at 212 nm and 400 nm. Aliquots of GNPs (10 µl diluted with 20 µl TFA, of which 20 µl was injected onto HPLC) were also analysed on HPLC to determine whether any free peptide remained in the GNP solutions, following centrifugation using the Amicon centrifugal filter tubes.

**Table 11. HPLC results summary**

| **ID** | **% insulin B (9-23)** | **% insulin B incorporation** | **% NPs with bound insulin B** | **Number of peptides/NP** |
|---|---|---|---|---|
| **NP151** | 1 | 98 | 98 | 3 |
| **NP152** | 2 | 98 | 98.5 | 5.9 |
| **NP153** | 1 | 45 | 91 | 1.4 |
| **NP154** | 2 | 55 | 98.5 | 3.3 |
| **NP157** | 0.5 | 32 | 92 | 0.47 |
| **NP158** | 0.5 | 61 | 98 | 0.92 |

### 1.4 Proinsulin C19-A3 Peptide

The aims of these experiments were to generate GNPs covalently conjugated to human proinsulin C19-A3 peptide having the amino acid sequence GSLQPLALEGSLQKRGIV (SEQ ID NO: 1), that all carry 1-2 peptides per GNP.

The synthesis strategy employed for producing C19-A3-carrying nanoparticles is depicted in Figure 1.

Initially, two types of C19-A3 conjugated GNPs were generated: NP51 (10% Glucose C2, 10% C19-A3) and NP52 (10% Mannose C2, 10% C19-A3) using a 75% methanol preparation method. HPLC analysis of peptide released from the C19-A3-GNPs following treatment with KCN solution (100 mM) found that the % of GNPs carrying peptide was 24.6% (NP51) and 20% (NP52), with 2.5 and 1.3 peptides bound per GNP for NP51 and NP52, respectively. However, incorporation efficiency of 10% C19-A3 peptide into these GNPs was low (5% for NP52 and 3% for NP51). TEM size distribution analysis for NP51 and NP52 confirmed that the majority of these GNPs were between 1.2 - 4.8 nm in diameter.

Given the low incorporation of 10% C19-A3 peptide into GNPs (3-5%) using the 75% methanol-based preparation method, solubility tests were performed to determine the optimal solution for C19-A3 peptide conjugation to gold nanoparticles. C19-A3 peptide was found to be highly soluble in water. Therefore, water-based GNP synthesis methods were developed using low (10%) or intermediate (35%) levels of methanol, and vivaspin centrifugation to remove any unbound peptide. GNPs containing 10% C19-A3 peptide and 10% glucose C2 (NP58) or 10% mannose C2 (NP61) were synthesised using a water-based (10% methanol) method (Table 12). HPLC analysis of peptide released from the C19-A3-GNPs following treatment with KCN solution (100 mM), confirmed 100% and 62% incorporation efficiency of C19-A3 peptide, with 31 and 19 peptides bound per GNP, for NP58 and NP61, respectively. An intermediate 35% methanol preparation method was also tested for synthesis of 10% C19-A3, 10% glucose C2 GNPs (NP63). HPLC analysis again confirmed high incorporation of C19-A3 peptide (67%), with 20 peptides bound per GNP.

Furthermore, lower amounts of C19-A3 peptide (1% (NP64), 0.5% (NP65), 0.1% (NP66), and 0.01% (NP67)) were conjugated to 10% glucose C2 GNPs using the 10% methanol preparation method to determine the level of C19-A3 peptide required to generate GNPs, that all carry 1-2 peptides per NP. HPLC analysis of these GNPs following treatment with KCN solution (100 mM) to release bound peptide, confirmed 55%, 56% and 100% peptide incorporation, with 1.7, 0.8 and 0.3 peptides bound per NP, for NP64, NP65, NP66, respectively (Table 12). The 0.01% peptide (NP67) was below detection limit of HPLC and was excluded. It was concluded that a 1% C19-A3 peptide concentration was optimal for the synthesis of C19-A3-GNPs that all carry 1-2 peptides/GNP.

Similar results were found for 10% mannose C2 GNPs conjugated to 1% (NP68), 0.5% (NP69) or 0.1% (NP70) C19-A3 peptide, with high peptide incorporation of 73%, 66% and 100%, respectively and 2, 1 and 0.3 peptides bound per GNP, respectively. The proportion of these GNPs carrying at least 1 peptide was 96%, 85% and 50% for NP68, NP69 and NP70, respectively. GNPs conjugated with 3% C19-A3 peptide and 10% glucose C2 (NP73) or 10% mannose C2 (NP74) were also synthesised using the 10% methanol preparation method to generate C19-A3-GNPs with an intermediate number of peptides/GNP. Incorporation efficiency of C19-A3 peptide was 93% for NP73 (10% glucose C2) and 54% for NP74 (10% mannose C2), with 8.4 and 5 peptides bound per GNP (Table 12).

In summary, a range of 10% glucose C2 and 10% mannose C2 GNPs, conjugated with different amounts of proinsulin C19-A3 peptide (10%, 3%, 2%, 1%, 0.5% or 0.1%) have been synthesised to generated C19-A3-GNPs loaded with high, medium or low numbers of peptides/GNP. Control GNPs with 10% glucose C2 (NP81) or 10% mannose C2 (NP82) and glutathione only GNPs (NP89) have also been synthesised (Table 12).

**Table 12: C19-A3 conjugated GNPs synthesised with different amounts of C19-A3 peptide using the 10% methanol-based method and control GNPs**

| **ID** | **% C19-A3 peptide** | **% GSH** | **% glucos e C2** | **% mannos e C2** | **µg C19-A3 incorporated/ml** | **% C19-A3 incorporation** | **% NPs with peptide** | **No. peptides /NP** |
|---|---|---|---|---|---|---|---|---|
| **NP58*** | 10 | 80 | 10 | - | 5970 | 100 | 95 | 31 |
| **NP61*** | 10 | 80 | - | 10 | 3656 | 62.3 | 96 | 19 |
| **NP63** | 10 | 80 | 10 | - | 3930 | 67 | 100 | 20 |
| **NP64*** | 1 | 89 | 10 | - | 324 | 55.3 | 97 | 1. 66 |
| **NP65** | 0.5 | 89. 5 | 10 | - | 165 | 56.3 | 80 | 0.84 |
| **NP66** | 0.1 | 89. 9 | 10 | - | 65 | 111 | 45 | 0.33 |
| **NP67** | 0.01 | 89. 99 | 10 | - | *below detection sensitivity | - | - | - |
| **NP68*** | 1 | 89 | - | 10 | 425 | 72.6 | 96 | 2.17 |
| **NP69** | 0.5 | 89. 5 | - | 10 | 192 | 66 | 85 | 1 |
| **NP70** | 0.1 | 89. 9 | - | 10 | 58 | 100 | 50 | 0.3 |
| **NP71** | 2 | 88 | - | 10 | 472 | 40.3 | 98 | 2.4 |
| **NP72** | 2 | 88 | 10 | - | 543 | 46 | 99 | 2.8 |
| **NP73*** | 3 | 87 | 10 | - | 1640 | 93 | 98 | 8.4 |
| **NP74*** | 3 | 87 | - | 10 | 955 | 54 | 99 | 5 |
| **NP81*** | - | 90 | 10 | - | - | - | - | - |
| **NP82*** | - | 90 | - | 10 | - | - | - | - |
| **NP89*** | - | 100 | - | - | - | - | - | - |

Proinsulin C19-A3 peptide-carrying nanoparticles were produced with the following properties (see Figure 1):
- C19-A3 peptide with a thiol proprionic acid linker in amide linkage at the N-terminus. This linker enables covalent binding of the peptide to gold via a S-Au bond.
- L-glutathione (oxidised) used as passivating agent, and to add negative charge (important for IL-10 binding).
- Glucose C2 (or mannose C2) - aids solubility. Mannose C2 tested to determine whether this enhances peptide-GNP uptake by dendritic cells (DCs).

Figure 2 shows a summary of the results of proinsulin C19-A3 peptide binding. Figure 2A shows the percentage of gold nanoparticles bound with C19-A3 peptide when nanoparticles are passivated with either 10% glucose or 10% mannose. Figure 2B shows the number of peptides bound per gold nanoparticle when nanoparticles are passivated with either 10% glucose C2 or 10% mannose C2.

Scaled-up production of C19-A3-GNP was performed using the Atlas Potassium automated synthesis system for toxicology studies.
- Product formulation: 3% C19-A3 peptide, 5% glucose C2 and 92% glutathione.
- Reaction in 100% water.
- Anti-foaming agent required to inhibit peptide foaming (0.025% 1-octanol for 500 ml scale in reduced reaction volume (335 ml)).
- Purification using 10 kDa amicon centrifugal filter tubes.
- Sterile filtration of product using 0.22 µm filter.

A number of analytics were used to QC batches of C19-A3 GNP:
- Quantification of peptide incorporation by HPLC
- Ligand ratio estimation by NMR
- TEM (Figure 5: TEM size analysis of 3% C19-A3 GNP toxicology batch).
- DLS size analysis and zeta potential
- MALDI-TOF analysis of peptide integrity (Figure 6)

### 1.5 Ovalbumin (323-339) Peptide

### Summary

The aims of this experiment were to generate GNP conjugated to 1%, 3% or 5% ovalbumin peptide (323-339) for ongoing murine studies.

### Methods

Ovalbumin 323-339 peptide [-S(CH₂)₂- CONH-ISQAVHAAHAEINEAGR]2 (SEQ ID NO: 11) was dissolved in DMSO (10 mg/ml). GNP with a glucose C2 (5%) and glutathione (94%, 92%, 90%) corona were covalently conjugated to ovalbumin peptide (1%, 3% or 5%)) using the water-based synthesis method. The nanoparticles were centrifuged to remove unbound peptide and DMSO using 10 kDa Amicon centrifugal filter tubes. The 3% and 5% ovalbumin-GNP underwent further filtration using 0.2 µm filter to remove unbound peptide. Peptide incorporation was determined using HPLC, following the addition of potassium cyanide solution (KCN) to disintegrate the GNP. Nanoparticle size and zeta potential were determined using dynamic light scattering (DLS).

### Results

HPLC analysis of peptide incorporation and colorimetric measurement of gold content found that 1% ovalbumin-GNP (NP299) had 1.3 peptides per GNP, with 90% of GNP carrying peptide. By HPLC, the 3% ovalbumin-GNP (NP302) were found to have 11% free peptide remaining after centrifugation using the 10 kDa filter tubes. Therefore, an additional filtration step using a 0.2 µm filter was included, which removed all of the free peptide. The filtered 3% ovalbumin-GNP (NP302) had 3.7 peptides per GNP, with 95% GNP carrying peptide. For the 5% ovalbumin-GNP (NP304), 6% free peptide remained in the preparation despite an additional 3 filtration steps using a 0.2 µm filter. This preparation had 5 peptides/GNP, with 98% of GNP carrying peptide. The DLS nanoparticle size of NP299 was ∼6.6 nm (by volume in PBS) and ∼6.2 nm (by volume in water), and for NP301, nanoparticle size was 6.5 nm (by volume in PBS) and ∼15 nm (by volume in water). For NP304, the GNP size was 10.2 nm (by volume in PBS) and 21 nm (by volume in water). The zeta potential of the nanoparticles was -44 mV, -48 mV and -59 mV for NP299, NP301 and NP304, respectively, confirming that the nanoparticles were very stable in water and well dispersed.

### Conclusions

GNP with higher ovalbumin peptide concentration >3%, are limited by difficulties in removing unbound peptide from the GNP preparations. Therefore, the 3% ovalbumin-GNP may be optimal for future electrostatic binding to human rIL-10.

### Methods

Ovalbumin 323-339 peptide [-S(CH₂)₂- CONH-ISQAVHAAHAEINEAGR]₂_(SEQ ID NO: 6) (AmbioPharm, Inc., SC, USA) was synthesised with a thiol proprionic acid linker in an amide linkage at the N terminal. For conjugation to GNP, ovalbumin 323-339 peptide first was dissolved in DMSO (10 mg/ml). 5% glucose C2 GNP conjugated to ovalbumin peptide (1%) were prepared using the 10% methanol (water-based) preparation method, using vivaspin centrifuge tubes to remove unbound peptide and DMSO. For all reactions, GNP were prepared by mixing 10 pmol gold with a 3 fold excess of glucose C2 or mannose C2 and glutathione ligands. Sodium borohydride (200 pmol; 7.6 mg in 200 µl water) was then quickly added to the reaction tubes in one go with continuous vortexing for 1 min. The reaction tubes were then incubated for 1 h at room temperature with constant mixing. After 1 h, the GNP solutions were transferred to Amicon centrifugal filter tubes (Millipore Ltd, 10 K membrane molecular weight cut-off) and the volume was made up to 4 ml by addition of 2 ml water. The GNP samples were centrifuged at 5000 rpm for 10 min to remove unbound ovalbumin peptide and DMSO. The supernatant was removed and stored at 4°C. The concentrated NPs on top of the membrane were then washed 4 times with water (4 ml) by centrifuging at 5000 rpm for 10 min each time. Water (1 ml) was added to the concentrated NPs and the NP solutions stored at 4°C until analysed. Note. For NP301 and NP304 additional purification using 0.2 µm filter was required to minimise the free peptide remaining in the GNP preparations. A gold assay was performed by colorimetric to determine gold content of ovalbumin peptide conjugated to the glucose or mannose C2 GNP (Table 1). DLS was performed to determine NP size (in 35% PBS solution) of ovalbumin peptide conjugated GNP (Table 13).

**Table 13: DLS size results for 5% glucose or mannose C2 GNP, conjugated with 1% or 0.5% ovalbumin**

| | **% ovalbumin** | **Gold content (mg/ml)** | **Mean size by vol. in water (nm)** | **Mean size by vol. in PBS (nm)** | **Zeta potential (mV)** |
|---|---|---|---|---|---|
| **NP299** | 1 | 1. 92 | 6.2 ± 3.0 | 6.6 ± 1.9 | -43.6 ± 0.7 |
| **NP301** | 3 | 1.69 | 15.0 ± 3.9 | 6.5 ± 3.4 | -47.9 ± 0.9 |
| **NP304** | 5 | 1.56 | 21.1 ± 18.9 | 10.2 ± 2.5 | -58.9 ± 1.0 |

### HPLC methods and results

Ovalbumin standard (2 µg; 20 µl) was injected for HPLC analysis and served as a reference standard. To determine the amount of ovalbumin peptide incorporated into GNP, 40 µl of 100 mM KCN in 10 mM KOH was added to 10 µl GNP, of which 20 µl was injected for HPLC analysis. HPLC chromatograms were acquired at 212 nm and 400 nm. Aliquots of GNP (10 µl diluted with 20 µl TFA, of which 20 µl was injected onto HPLC) were also analysed on HPLC to determine whether any free peptide remained in the GNP solutions, following centrifugation using the Amicon centrifugal filter tubes.

**Table 14: HPLC Results Summary**

| **ID** | **% ovalbumin** | **Filtration 0.2 um filter** | **% ovalbumin incorporated** | **Total peptide released by KCN (µg/ml)** | **Total free peptide (µg/ml)** | **% free peptide** | **Peptide incorporation in GNP (µg/ml)** | **% NPs with bound ovalbumin** | **Number of bound peptides/NP** |
|---|---|---|---|---|---|---|---|---|---|
| NP299 | 1 | N | 41 | 231.5 | 0 | 0 | 231.5 | 90 | 1.3 |
| NP301 | 3 | N | 39 | 774.7 | 85.5 | 11 | 689.2 | 97 | 4.3 |
| NP301 | 3 | Y | 38 | 630.5 | 0 | 0 | 630.5 | 95 | 3.7 |
| NP304 | 5 | Y | 29 | 806.3 | 47.7 | 6 | 757 | 98 | 5 |

### Example 2 - Optimisation of electrostatic binding of human recombinant interleukin-10 (IL-10) to proinsulin C19-A3 peptide conjugated gold nanoparticles

The aim of this experiment was to investigate electrostatic binding of a tolerance-enhancing element (human recombinant IL-10) to proinsulin C19-A3 peptide-carrying nanoparticles as prepared in Example 1, section 1.4.

Initially, two types of C19-A3 peptide conjugated GNPs were generated: NP51 (10% Glucose C2, 10% C19-A3) and NP52 (10% Mannose C2, 10% C19-A3) using a 75% methanol preparation method. However, incorporation efficiency of 10% C19-A3 peptide into these GNPs was low (5% for NP52 and 3% for NP51). TEM size distribution analysis for NP51 and NP52 confirmed that the majority of these GNPs were between 1.2 - 4.8 nm in diameter.

Binding studies, using myoglobin (from equine skeletal muscle) as a surrogate for human recombinant IL-10, as it has similar pI and molecular weight (Mol. Wt. = 17.6 kDa; pI: 7.3), were performed to test binding efficiencies to GNPs. Binding of myoglobin to control 10% glucose GNPs (NP40) using range of sodium acetate-acetic acid pH buffers (0.1 M or 0.05 M) showed that pH 4.6, 0.05 M sodium acetate-acetic acid buffer was optimal for electrostatic binding to myoglobin (Figure 3). Electrostatic binding of myoglobin (40 µg) to different amounts of NP51 and NP52 using pH 4.6, 0.05 M buffer, found 92-100% myoglobin binding using 8, 10, 12 or 16 µl NP, with 100% binding to the NPs using 16 µl NP.

### Generation of peptide-IL-10 coupled GNPs

Human recombinant IL-10 was obtained from Cell Guidance Systems, Cambridge (Mol. Wt. = 16.6 kDa; pI = 7.65). Since rIL-10 is supplied freeze-dried in NaP, the rIL-10 (100 µg) was dialysed into sodium acetate-acetic acid buffer (pH 4.6 0.05 M). A range of dialysis systems were tested for recovery yield of rIL-10. The Pur-A-lyser Midi 3500 microdialyser (3.5 kDa cut-off) (Sigma) was found to be the most efficient system, with 84% of rIL-10 recovered post- dialysis. Microdialysis of human recombinant IL-10 into sodium acetate-acetic acid buffer (pH 4.6, 0.05 M) enabled electrostatic binding of human rIL-10 to C19-A3 GNPs, with 60% human rIL-10 bound to NP51 (10% Glucose C2 (NP55)) and 90% bound to NP52 (10% Mannose C2 (NP56)) (Table 15). NP51, NP52 and rIL-10-C19-A3-GNPs (NP55 and NP56) were used for *in vitro* testing. Human rIL-10+GNPs without any peptide (90% Glutathione, 10% Glucose C2) (NP57) were also prepared as above. 83% human rIL-10 bound to NP57.

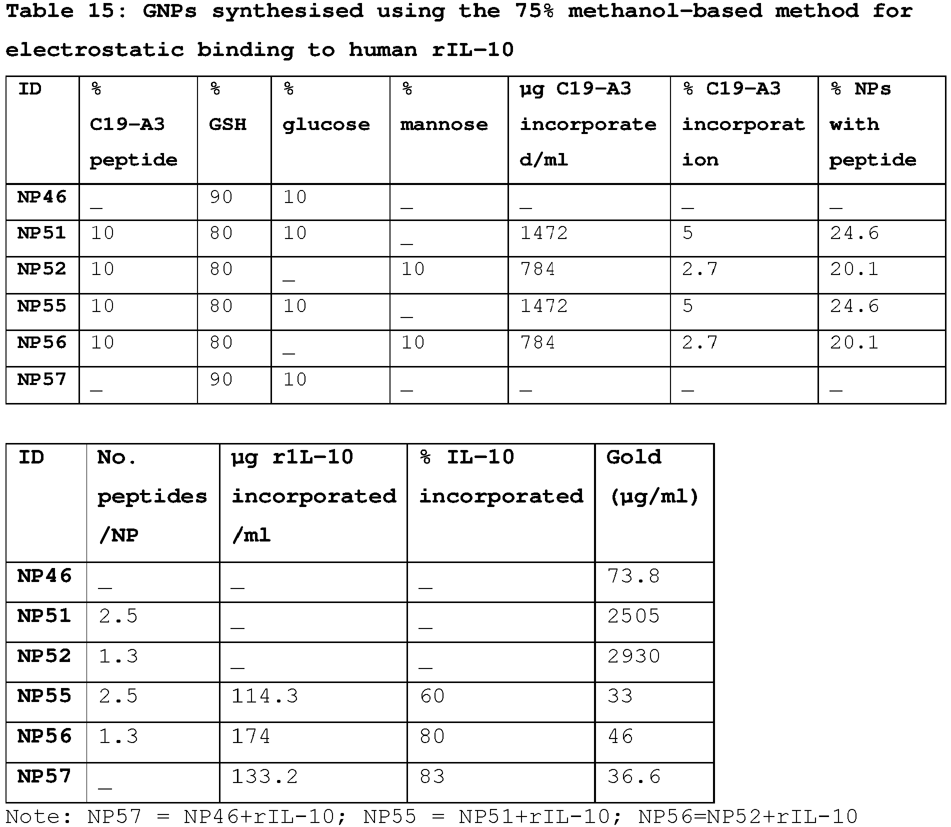

Given the low incorporation of 10% C19-A3 peptide into GNPs (3-5%) using the 75% methanol-based preparation method (see Example 1), solubility tests were performed to determine the optimal solution for C19-A3 peptide conjugation to gold nanoparticles. C19-A3 peptide was found to be highly soluble in water. Therefore, water-based GNP synthesis methods were developed using low (10%) or intermediate (35%) levels of methanol, and vivaspin centrifugation to remove any unbound peptide. GNPs containing 10% C19-A3 peptide and 10% glucose C2 (NP58) or 10% mannose C2 (NP61) were synthesised using a water-based (10% methanol) method. HPLC analysis of peptide released from the C19-A3 GNPs following treatment with KCN solution (100 mM), confirmed 100% and 62% incorporation efficiency of C19-A3 peptide, with 31 and 19 peptides bound per GNP, for NP58 and NP61, respectively. An intermediate 35% methanol preparation method was also tested for synthesis of 10% C19-A3, 10% glucose C2 GNPs (NP63). HPLC analysis again confirmed high incorporation of C19-A3 peptide (67%), with 20 peptides bound per GNP. However, binding tests using myoglobin as a surrogate for rIL-10, found that the high number of C19-A3 peptides carried per GNP significantly reduced the binding capacity of myoglobin to NP58, NP61 and NP63.

Therefore, lower amounts of C19-A3 peptide (1% (NP64), 0.5% (NP65), 0.1% (NP66), and 0.01% (NP67)) were conjugated to 10% glucose C2 GNPs using the 10% methanol preparation method to determine the level of C19-A3 peptide required to generate GNPs, that all carry 1-2 peptides per NP. HPLC analysis of these C19-A3 GNPs following treatment with KCN solution (100 mM) to release bound peptide, confirmed 55%, 56% and 100% peptide incorporation, with 1.7, 0.8 and 0.3 peptides bound per NP, for NP64, NP65, NP66, respectively. The 0.01% peptide (NP67) was below detection limit of HPLC and was excluded. It was concluded that a 1% C19-A3 peptide concentration was optimal for the synthesis of C19-A3 GNPs that all carry 1-2 peptides/GNP.

Binding studies of myoglobin to NP64, NP65 and NP66 confirmed that the lower number of C19-A3 peptides per GNP greatly improved myoglobin binding, with maximal binding efficiencies of 80-90%.

*Modification of 1% C19-A3 peptide conjugated GNPs with different levels of carbohydrate element, glucose C2 or mannose C2* GNPs conjugated with 1% C19-A3 peptide and variable amounts of glucose C2 or mannose C2 (5% (NP75, NP76), 20% (NP77, NP78), 40% (NP79, NP80) were synthesised to determine binding potential, using myoglobin as a surrogate for rIL-10. Control GNPs, with variable glucose C2 or mannose C2 were also prepared (5% (NP83, NP84), 20% (NP85, NP86), 40% (NP87, NP88) (Table 16). Myoglobin binding studies found that 5% or 10% glucose C2 ligand was optimal for binding to 1% C19-A3-glucose-GNPs while 5% mannose C2 was the optimal carbohydrate ligand concentration for myoglobin binding to 1% C19-A3-mannose-GNPs. To verify this finding, 1% C19-A3 peptide GNPs, containing 2.5%, 5%, 7.5%, and 10% or 12.5% glucose C2 or mannose C2 were then generated (Table 16). Myoglobin binding efficiencies to 1% C19-A3 GNPs containing 2.5-10% glucose C2 were similar (52-56% myoglobin binding) (Figure 4). By comparison, 2.5% or 5% mannose C2 was the optimal carbohydrate ligand concentration for myoglobin binding to 1% C19-A3-mannose-GNPs with reductions in myoglobin binding efficiency with higher mannose C2 concentrations.

To determine the optimal C19-A3 peptide concentration for electrostatic binding of myoglobin, a range of 5% glucose C2 and 5% mannose C2 GNPs, conjugated with variable amounts of C19-A3 peptide (0.1%, 0.5%, 1%, 3%, 10%) were generated (Table 16). Myoglobin binding efficiency to 5% mannose C2 GNPs, conjugated with 0.1%, 0.5% or 1% C19-A3 peptide were 34%, 35% and 51%, respectively but decreased to 22% and 5% with increased C19-A3 peptide concentrations of 3% and 10%, respectively. Myoglobin binding to 5% glucose C2 GNPs, conjugated with 0.1%, 0.5% or 1% C19-A3 peptide was 50%, 66% and 56% and decreased to 37% and 6% for glucose C2 GNPs, conjugated to 3% and 10% C19-A3 peptide, respectively.

The present results point to the advantageous use of electrostatic binding of human rIL-10 to C19-A3 GNPs using 1% C19-A3 peptide conjugated GNPs with 5% glucose C2 or 5% mannose C2, generated using the water-based (10% methanol) preparation method. In parallel, GNPs linked to IL-10 and non-GMP indicator peptides for which T cell clones are available e.g. HA and TT peptides (referred to below as "peptide-IL-10-NPs") are generated using this refined preparation method. Moreover, fluorescently labelled C19-A3 GNPs using 1% C19-A3 peptide conjugated GNPs with a range of glucose C2 or 5% mannose C2 ligand have also been generated.

**Table 16. 1% C19-A3 peptide conjugated GNPs synthesized with variable amounts of the carbohydrate element, glucose C2 or mannose C2, and control GNPs**

| **ID** | **% C19-A3 peptide** | **% GSH** | **% glucose C2** | **% mannose C2** | **µg C19-A3 incorporated/ml** | **% C19-A3 incorporation** | **% NPs with peptide** | **No. peptides /NP** |
|---|---|---|---|---|---|---|---|---|
| NP75 | 1 | 94 | 5 | - | 336.8 | 58 | 99 | 1.7 |
| NP76 | 1 | 94 | - | 5 | 298 | 51 | 98.5 | 1.5 |
| NP77 | 1 | 79 | 20 | - | 265 | 45.2 | 99 | 1.4 |
| NP78 | 1 | 79 | - | 20 | 470 | 80 | 73 | 2.4 |
| NP79 | 1 | 59 | 40 | - | 470 | 80 | 78 | 2.4 |
| NP80 | 1 | 59 | - | 40 | 290 | 50 | 78 | 1.5 |
| NP83 | - | 95 | 5 | - | - | - | - | - |
| NP84 | - | 95 | - | 5 | - | - | - | - |
| NP85 | - | 80 | 20 | - | - | - | - | - |
| NP86 | - | 80 | - | 20 | - | - | - | - |
| NP87 | - | 60 | 40 | - | - | - | - | - |
| NP88 | - | 60 | - | 40 | - | - | - | - |
| NP90 | 1 | 96.5 | 2.5 | | 412.5 | 70 | 88 | 2.11 |
| NP91 | 1 | 96.5 | - | 2.5 | 335 | 57 | 96 | 1.72 |
| NP92 | 1 | 91.5 | 7.5 | - | 285 | 49 | 92.5 | 1.46 |
| NP93 | 1 | 91.5 | - | 7.5 | 358 | 61 | 94 | 1.83 |
| NP94 | 1 | 86.5 | 12.5 | - | 260 | 52 | 89 | 2.17 |
| NP95 | 1 | 86.5 | - | 12.5 | 307.4 | 52 | 76 | 1.57 |
| NP98 | 0.5 | 94.5 | 5 | - | 275 | 94 | 95 | 1.4 |
| NP99 | 0.5 | 94.5 | - | 5 | 240 | 82 | 98 | 1.2 |
| NP100 | 3 | 92 | 5 | - | 932.8 | 53 | 100 | 5 |
| NP101 | 3 | 92 | - | 5 | 1060 | 60.3 | 100 | 5 |
| NP102 | 10 | 85 | 5 | - | 3074 | 52 | 100 | 16 |
| NP103 | 10 | 85 | - | 5 | 2597 | 44.3 | 100 | 13.3 |
| NP104 | 1 | 89 | - | 10 | 350 | 60 | 83 | 1.8 |

### Summary:

- Microdialysis of rIL-10 into pH 4.6 sodium acetate-acetic acid buffer required.
- 5% glucose C2 (or mannose C2) optimal sugar ligand concentration on GNP. >5% reduces binding efficiency.
- Achieve ~1:3 of rIL-10:C19-A3 for 1% C19-A3 GNP and ~1:8 ratio for 3% C19-A3 GNP (µg/ml GNP).
- rIL-10-peptide GNP soluble in Na borate buffer (pH 8.5, 0.05 M) but insoluble in water.

### Example 3 - Generation of human recombinant IL-10 gold nanoparticles (5% glucose C2 and 95% glutathione) (NP298)

IL-10-GNP were developed for use in murine models of type 1 diabetes, in order to determine whether single-cargo delivery of IL-10-GNP offers greater flexibility in achieving optimal dosing of IL-10 and C19-A3 peptide compared to the dual-cargo IL-10/C19-A3-peptide-GNP approach of Example 2. Single cargo IL-10-GNP can be delivered by microneedle injection either prior to C19-A3 peptide-GNP microneedle injection or simultaneous with peptide-GNP, in order to determine the optimal mode and timing of delivery.

### A) Microdialysis of rIL-10 (0.1 mg) using Pur-A-lyser Midi 3500 microdialyzer

Human rIL-10 (0.1 mg) (Cell Guidance Systems) was dissolved in 350 µl water by gentle mixing for 1 h. An aliquot of the pre-dialysed human rIL-10 (1.6 µg) was analysed by HPLC and served as a rIL-10 standard. The remaining human rIL-10 was transferred to a Pur-A-lyser 3500 Midi microdialyzer tube, and dialysed in 250 ml of Na acetate-acetic acid buffer (50 mM, pH 4.6) for 1 h at room temperature, with continuous stirring.

The area of pre-dialyzed rIL-10 standard 1.6 µg was determined as 14.692 mAu.min. The area of post-dialysed rIL-10 (5.6 µl) was determined as 16.309 mAu.min. This is 1.1 fold greater than the rIL-10 standard i.e. 1.76 µg in 5.6 µl. Therefore, 91 µg of human rIL-10 was recovered after dialysis into Na acetate-acetic acid buffer (50 mM, pH 4.6).

### B) Control GNP synthesis

For control-GNP preparations, 30 pmol gold was mixed with 5% glucose C2 and 95% glutathione (ligands mixed in a 3-fold excess to gold), and the reaction mixture made up to 6 ml with 75% methanol. Sodium borohydride (600 pmol; 22.8 mg in 600 µl water) was added to the reaction tubes with continuous vortexing for 1 min. The reaction tubes were then incubated for 1 h at room temperature with constant mixing. After 1 h, the solutions were transferred to Amicon centrifugal filter tubes (Millipore Ltd, 10 K membrane molecular weight cut-off) and centrifuged at 5000 rpm for 6 min to remove any unbound peptide. The supernatant was removed and stored at 4°C.

The concentrated nanoparticles present on top of the membrane were then washed 3 times to ensure complete removal of unbound peptide, by addition of 5 ml of water to the filter tubes followed by centrifugation at 5000 rpm for 6 min. The volume of concentrated GNP was made up to 3 ml with water and stored at 4°C. Gold content was determined by colorimetric assay and GNP size and zeta potential determined by dynamic light scattering (DLS).

### C) Electrostatic binding of rIL-10 (in pH 4.6 buffer) to GNP

Dialysed human rIL-10 was incubated with control GNP (5% glucose, 95% glutathione) as shown below.

**Volumes used for electrostatic binding of dialyzed rIL-10 to peptide conjugated GNP**

| **Control-GNP ID** | **Dialysed rIL-10 (µg)** | **GNP (µl)** | **rIL-10+peptide-GNP new ID** |
|---|---|---|---|
| NP296 | 91 | 42 | NP298 |

Samples were incubated for 1 h at room temperature with continuous mixing on a rocker. After 1 h, the samples were centrifuged at 5,000 rpm for 5 min. The supernatant was removed and the GNP pellet resuspended in pH 8.5 Na borate buffer (0.05 M) (900 µl).

### D) Analytical methods and results

HPLC analysis was performed at 212 nm and 400 nm using a Varian 900-LC, with a reverse phase C18 column (Acquisition time: 11.2 min; Temperature = 35oC; Slit width = 2 nm; 95% water, 5% acetonitrile gradient (1 ml/min) switching at 8 min, switch to 20% water, 80% acetonitrile). A gold colorimetric assay was performed to determine the gold content in the rIL-10-GNP and control-GNP. Zeta potential and DLS size measurements were performed using the Zetasizer Nano-ZSP (Malvern) and analysed using the Zetasize software (version 7.01)

**Table 17: HPLC results summary**

| **GNP ID** | **% rIL-10 incorporated** | **µg rIL-10 bound to GNP** | **IL-10-GNP (µl)** | **µg rIL-10 /ml GNP** | **No. rIL-10 /GNP** |
|---|---|---|---|---|---|
| NP298 | 82 | 72.4 | 900 | 82.4 | 2.6 |

**Table 18: Gold content, DLS size (by volume) and zeta potential of rIL-10-GNP (NP298) and control-GNP (NP296)**

| | **Gold content (mg/ml)** | **Mean size in water (by vol.) (nm)** | **Mean size in PBS (by vol.) (nm)** | **Zeta potential (mV)** |
|---|---|---|---|---|
| **NP296** | 2.0 | 26.5 ± 2.7 | 3.3 ± 0.6 | -57.5 ± 2.6 |
| **NP298** | 0.038 | 26.8 ± 7.3 | 0.7 ± 0.0 | -38.4 ± 3.8 |
| **NP298 snat** | 0.106 | | | +60.5 ± 12.7 |

By HPLC, IL-10-GNP (NP298) had ~2.6 IL-10 bound per GNP (82.4 µg IL-10/ ml GNP), with 82% of IL-10 incorporated. Zeta potential analysis found that IL-10-GNP were less negatively charged (-38 mV) compared to control-GNP, without IL-10 (-58 mV), while the supernatant containing any remaining free IL-10 and unbound GNP had a very positive charge of +60 mV. The DLS nanoparticle size of control-GNP (NP296) was 3 nm in PBS and 26 nm in water, and was similar for IL-10-GNP (0.7 nm in PBS and 27 nm in water).

### Example 4 - Intradermal administration of antigen coupled to 2-20 nm gold nanoparticles is a means of inducing antigen specific tolerance

The immune system is generally tolerant to self-proteins, reserving its immune response for foreign antigens, microbes, and tumours. This balance is maintained by several mechanisms including thymic deletion of self-reactive T cells (central tolerance) and the generation of self-antigen specific regulatory T cells (Treg) which downregulate reactions to self-proteins.

Organ-specific autoimmune diseases, such as type 1 diabetes, multiple sclerosis, primary biliary cirrhosis, myasthenia gravis and autoimmune thyroid disease, occur when this system fails and damaging immune responses to self-proteins arise. Current therapies for such conditions include drugs causing generalized immunosuppression, but while effective, these agents expose the patient to increased risks from infection and malignancies. A therapeutic system capable of restoring tolerance to the self-antigens relevant to a particular autoimmune disease without causing generalized immunosuppression is highly desirable. One potential means of achieving this is to induce Tregs specific for one or more of the antigens derived from the target organ of the autoimmune disease. Such a therapeutic might be termed a "tolerogenic vaccine".

Antigens are only recognised by the immune system if they are initially presented by antigen presenting cells (APCs, such as a dendritic cells) to T cells (T lymphocytes). It has previously been shown that antigens presented to the immune system by resting or uninflamed APCs tends to induce tolerance and Treg formation, and that this process occurs continuously for self-proteins. Features of APCs that are able to induce tolerance (tolerogenic APCs) include low expression of costimulatory molecules, relative low expression of HLA-class II molecules, low expression of inflammatory cytokines such as IL-12, and increased expression of regulatory cytokines such as IL-10.

Here we show that intradermal administration of antigens complexed to very small gold nanoparticles and/or co-administered with IL-10 coupled to gold nanoparticles represents an appropriate means of inducing antigen-specific tolerance, as it delivers antigen effectively to APCs while promoting their un-inflamed or "immature" state.

### GNPs do not induce maturation of APCs or reduce pro-inflammatory cytokine expression when APCs are exposed to an inflammatory stimulus.

Human immature monocyte derived dendritic cells (iDCs) were incubated with proinsulin C19-A3 coupled GNPs or without GNPs for 4 h. Thereafter, the GNPs were washed off and cells left overnight, either alone or in the presence of 100ng/ml LPS. Overnight maturation of DCs was measured as increases of CD80 and HLA-DR expression using FACS.

The uptake of the proinsulin C19-A3 GNPs alone did not induce spontaneous pro-inflammatory differentiation of iDCs, nor did it prevent subsequent maturation of DCs with a TLR-ligand LPS (a toll-like receptor-ligand lipopolysaccharide) with respect to phenotype change (Figure 7A). LPS induced up-regulation of MHC-class II and co-stimulatory molecules to a similar level in both proinsulin C19-A3 GNP treated and non-GNP treated mature DCs (Figure 7A).

iDCs were also incubated with proinsulin C19-A3 GNPs coupled to glucose (GNPgluc), with proinsulin C19-A3 GNPs coupled to mannose (GNPman), or without GNPs for 4hrs. Thereafter, cells were washed and subsequently stimulated with 100ng/ml LPS and left overnight. Supernatants were collected the next day and cytokine (IL-10, IL-12 and TNF) release by DCs measured using Luminex.

Unlike LPS-only treated DCs, LPS and proinsulin C19-A3 GNP-treated DCs showed reduced production of cytokines, indicating that treatment with proinsulin C19-A3 GNPs changed the maturation capacity of iDCs (Figure 7B).

### GNPs coupled to antigen are taken up and presented by antigen presenting cells efficiently

iDCs were incubated with either soluble proinsulin C19-A3 peptide, proinsulin C19-A3-coupled GNPs (C19-A3-GNP), or GNPs alone (no pep-GNPs) for 4hrs, after which free NPs were washed off and DCs matured with LPS for 48hrs. Mature DCs were subsequently incubated overnight with a proinsulin C19-A3-specific Treg clone that produces IL-10 in response to cognate reaction with DCs. IL-10 concentration in the supernatant was measured using Luminex.

Figure 8 indicates that human DCs present proinsulin peptide C19-A3 GNPs to T cells. Similar data has been obtained in a murine system; Figure 9 shows presentation of the BDC2.5 mimotope peptide recognised by BDC-2.5 clonal T cells in mouse spleen cells from BDC-2.5 transgenic mice in vitro. A 3 amino-acid "linker" was added to the peptide to allow it to be covalently attached to the GNPs.

### GNPs administered intradermally reflux back into the upper dermis and epidermis where there are large numbers of APCs

Intradermal injection of 30 nm latex nanoparticles into human skin deposits the majority of nanoparticle material into the upper layers of the dermis, as shown in Figure 10. However, following injection of GNPs (1-10 nm), a significant amount of material is detectable in the epidermis, where there are large numbers of APCs including Langerhans cells (CD207 positive), as shown by histology (Figure 11) and flow cytometry (Figure 12).

### GNPs coupled to antigen are transported to draining LNs via migratory DCs

PBS, ovalbumin peptide (323-339), or ovalbumin peptide (323-339) coupled GNPs (NP167, NP168) were administered intradermally to C57BL-6 mice, at the same time as i.v. administration of fluorescent cell trace violet (CTV) labelled OT-II (Ovalbumin peptide specific) CD4 positive T cells. Draining LN and spleen cells were harvested 3 days after ovalbumin (323-339) peptide administration, and studied by flow cytometry. Where fluorescent cells are seen but the level of fluorescence has been reduced, this implies that they have recognised their target peptide on APCs in that tissue and undergone cell division. The design of the experimental system is shown in Figure 13.

Figure 14 shows that GNPs are as, or more effective than, ovalbumin peptide alone at delivering ovalbumin (323-339) peptide to T cells in the draining LN. Injection of PBS or peptide alone results in low levels of accumulation and proliferation, whereas much higher levels are seen with ovalbumin peptide (323-339) bearing NPs.

In order to determine whether the injected peptide is carried to draining LNs in fluid phase or carried via migratory skin DCs, homozygous knockout mice for the chemokine receptor CCR7 (which mediates DC migration from skin) were treated in the same protocol as above.

Figure 15 shows that reduced numbers of fluorescent cells are accumulated in the LN following intradermal injection of ovalbumin (323-339) GNPs in CCR7-/- mice, suggesting that ovalbumin (323-339) peptide GNPs are transferred to the LN at least in part via migratory DCs.

### GNPs coupled with antigen administered intradermally disseminates more rapidly and widely in the immune system than antigen alone

A similar in vivo protocol was designed, using fluorescently labelled indicator CD4⁺ T cells (BDC-2.5, specific for chromograninA 342-355 found in pancreatic beta cells) and the peptide "BDC-2.5 mimotope" (a peptide of similar configuration to this epitope, that mimics the chromogranin A 342-355 peptide) coupled to GNPs linked to either mannose C2 or glucose C2.

Figure 16 shows that injection of BDC-2.5 mimotope peptide alone produces a modest accumulation of proliferating indicator cells in comparison to PBS in draining LN (axillary LN). However, GNP linked to BDC-2.5 mimotope peptide at the same concentration produces a more marked response, and this response is seen not just in the draining LN, but in distant LNs (inguinal, para-aortic) and spleen.

In these experiments, proliferation of indicator cells is seen in the pancreatic LN (PLN) even in the absence of injected antigen (PBS). This is because the beta cells of the pancreas contain the target epitope and it is therefore presented constitutively in the PLN. However, administration of GNP linked to BDC-2.5 mimotope peptide (but not peptide alone) results in a further increase in proliferation in the PLN, suggesting that the GNP carries the peptide to this location also, as seen for other LNs distant from the injection site. This is likely to be highly relevant to treatments aimed at targeting the response in the pancreas.

### Example 5 - Cytokines coupled to GNPs disseminate widely in the immune system; tolerogenic cytokines given in this way have the potential to promote tolerance induction.

Data from Example 4 suggests that intradermally administered antigen coupled to GNPs is less inflammatory and disseminates more widely that antigen alone. As a means of promoting the induction of antigen specific tolerance further, it is desirable to give a second signal, along with the antigen, that promotes induction of Tregs.

Here we present evidence that coupling of the tolerogenic cytokine IL-10 to GNPs reduces expression of costimulatory molecules by DCs following inflammatory stimuli (promoting an immature, pro-tolerogenic phenotype), as well as reducing production of inflammatory cytokines.

### IL-10 co-administered or coupled to GNPs reduces DC maturation and pro-inflammatory cytokine production on exposure to inflammatory stimuli

Immature DCs were treated either with soluble IL-10, proinsulin C19-A3 GNPs, or proinsulin C19-A3 GNPs coupled to IL-10 and subsequently either left alone or additionally stimulated with LPS. iDC incubation with IL-10 coupled GNPs did not produce a significant effect on the immature DC phenotype, compared with peptide-only-carrying GNPs (Figure 17A, upper row). However, IL-10-GNPs changed the ability of DCs to upregulate HLA-DR and activation molecules CD80 and CD25 upon stimulation with LPS. Resulting LPS-treated DCs incubated with soluble IL-10 or IL-10 coupled to GNPs had an immature phenotype (Figure 17A, lower row), and also reduced pro-inflammatory cytokine production (IL-12 and TNF) (Figure 17B).

These data suggest significant changes in the antigen-presenting capacity of DCs following treatment with peptide-GNPs coupled to IL-10. Of note, IL-10's effect was similar (in cell culture) when added as soluble protein or coupled to GNPs. In addition, DCs treated with soluble IL-10 or peptide-IL-10-GNPs (final IL-10 concentration 100 µg/ml), showed significantly higher release of IL-10 in the culture, and less production. Additional experiments have shown an increase in IL-10 mRNA, indicating that at least part of this increase in IL-10 is generated by the DCs themselves. This increase in local IL-10 concentration is expected to enhance regulatory T cell induction upon subsequent interaction between the DCs and T cells.

### Co-delivery of peptide and IL-10 results in reduced antigen specific proliferation

The efficacy of peptide presentation by GNPs to pro-inflammatory T cells was tested using human glutamic acid decarboxylase (GAD) peptide (339-352) specific T cell clones (generated by LUMC). These cells were used to test the possible effects of IL-10 on specific immune stimulation of peptide specific regulator T cells.

As the proinsulin peptide C19-A3 specific T cell clones that we have available are already of regulatory phenotype, Th1-type T cells specific for a different diabetes-linked peptide epitope GAD (339-352) were used. To that end, 1 % GAD peptide (339-352) coupled GNPs were prepared with either 5% glucose or 5% mannose. Human rIL-10 was also incorporated into a portion of the GAD-GNP preparations reaching 100% efficiency of incorporation of IL-10 at an approximate ratio of 1:10 IL-10 to GAD peptide.

Monocyte derived dendritic cells (moDCs) of HLA type DR17 were used as stimulator cells and a CD4+ T-cell clone specific for GAD (339-352) restricted by HLA-DR17 were the effector cells. After incubation of moDCs with the GNP constructs and subsequent maturation induced by LPS, DCs were collected, thoroughly washed and incubated with CFSE-labelled GAD-specific T cells. Cell proliferation was determined after 3-day co-culture.

Results show that the moDCs take up the GAD-GNP, process, and present the GAD 339-352 peptide as efficiently as previously shown for the proinsulin C19-A3 peptide. The GAD peptide was presented equally well upon delivery with either glucose- or mannose-containing GNPs, and both constructs induced T cell proliferation equally well and to a similar magnitude as free peptide (Figure 18A). Passage of the GNP solution through microneedles had no influence on the bio-potency of the GAD-GNP constructs, demonstrating that, in all likelihood, the choice of GNP administration would not interfere with the anticipated immune stimulation in patients (Figure 18B).

Finally, the effects of addition of IL-10 to the system were investigated. It was found, not unexpectedly, that the addition of IL-10 to the GAD-GNP (either with glucose or mannose) and subsequent testing using the same protocol as described above, clearly showed reduced DC maturation (Figure 17) and suppressed antigen presentation measured by reduced T cell proliferation (Figure 18C). This confirms that the IL-10 delivered on GNPs (as part of a dual cargo with antigenic peptide) is biologically active and generates levels of IL-10 that have effects on T cell function.

### IL-10 coupled to GNPs delivered intradermally disseminates more widely than IL-10 alone, tracking with the delivery of antigen to distant LNs.

A preliminary *in vivo* experiment was carried out using the murine BDC-2.5 mimotope peptide and fluorescently-labelled indicator CD4 T cell clones specific for this peptide described above. GNPs were given coupled to peptide (BDC-2.5 mimotope), IL-10, or both, and compared to blank GNPs of injected controls. Free IL-10 had no effect of proliferation, whereas IL-10 at the same concentration coupled directly to the BDC-2.5 mimotope peptide carrying GNP resulted in downregulation of proliferation not only in the draining LN (axiliary LN) but at all sites. This provides evidence that the cytokine travels with the GNPs to distant sites when complexed with GNPs, and in bioactive form. This is of enhanced efficiency compared to IL-10 alone.

### Example 6 - GNP-coupled IL-10 induces de novo IL-10 synthesis and suppressive capacity in T cells

Immature human dendritic cells (huDC) were incubated with recombinant IL-10, GNP_{c19-a3} alone or coupled to IL-10 for 4 hours, after which cells were washed and medium refreshed containing GM-CSF alone (800 U/ml) or combined with 100ng/ml LPS. IL-10 added to the cultures (alone or on GNP) was set to 100ng/ml. After 18hrs of culture, cells were washed, mRNA isolated and measured using RT-PCR. Relative expression was calculated using a standard housekeeping gene OAZ1. Figure 19 shows that GNPs coupled to IL-10 induce *de-novo* synthesis of IL-10 mRNA in dendritic cells. This confirms that the dendritic cells were induced to make their own IL-10 by treatment with IL-10 coupled GNPs.

Immature huDC were incubated with recombinant GNP_{c19-a3} alone or coupled to IL-10 overnight, after which DCs were washed and used to stimulate autologous naive isolated CD4+ T cells. After a 2-week culture (ref. Unger et al. EJI 2009), cytokine production and suppressive capacity of remaining T cells were tested. Treg lines were stimulated with mature DCs pulsed with either c19-a3 peptide (relevant Ag) or GAD339-352 peptide (irrelevant Ag) and cytokine production after overnight culture was measured. Figure 20A demonstrates antigen-dependent increase in IL-10 production by T cells and a superior capacity to produce IL-10 by T cells stimulated with the IL-10-coupled GNP_{c19-a3}. Naive CD4+ cells exposed to DCs treated with GNP-IL-10 therefore make more IL-10.

Naive CD4+ responder cells isolated form a HLA-mismatched donor were labeled with Carboxyfluorescein succinimidyl ester (CFSE) and co-incubated with cells as described above. After a 3-day co-culture, cell proliferation (CFSE-dilution) was determined using Flow-Jo software. Figure 20B shows a strong decrease in proliferation of Tresponder cells when co-incubated with Tregs generated with GNP-treated DCs. These T cells have suppressive capacity - i.e. the endogenous IL-10 is a marker now of regulatory capacity.

These experiments therefore support the conclusion that the dual cargo GNP-C19-A3-IL-10 nanoparticles exhibit advantageous induction of a specific suppressive or regulatory T cell response to C19-A3.

### Example 7 - Analysis of GNP ligand percentages

The relative proportions or percentages of different covalently bound ligands may be expressed in terms of the starting materials used in the nanoparticle synthesis, e.g. a 10% glucose-C2:90% glutathione gold nanoparticle may indicate that the ligands thioethyl glucose and glutathione were present at a ratio of 1:9 when added to the gold salt solution in order to form the nanoparticle. However, in some cases the ratio of starting materials is not necessarily reflected directly in the ratio of ligands bound to the finished nanoparticle. For example, the finished nanoparticle prepared from 1:9 thioethyl glucose and glutathione may be analysed by one or more analytical techniques (e.g. HPLC and/or NMR) in order to determine the percentage or relative proportion of each ligand.

A batch of gold nanoparticles having covalently bound ligands of: C19-A3 peptide (via thiol propionic acid linker in amide linkage at the N-terminus of the peptide), thioethyl-glucose, and L-glutathione were prepared essentially as described in Example 1, section 1.4. The proportions of the ligand reactants used in the synthesis expressed as percentage by number were: C19-A3 peptide 3%; glucose-C2 5%; GSH 92%. The ligand proportions by number on the final nanoparticles were then determined by NMR. The results were as follows:
Average number/nanoparticle

| | |
|---|---|
| C19-A3 peptide: | 3.6 |
| Glucose-C2: | 12 |
| Glutathione: | 29 |

Assuming 44 ligands/nanoparticle, the NMR data imply the following percentages by number:

| | |
|---|---|
| C19-A3 peptide: | 8.2% |
| Glucose-C2: | 27.3% |
| Glutathione: | 65.9% |

It is apparent that the percentage of glutathione is lower than would be expected based on the reaction input proportions, while that of C19-A3 peptide and glucose-C2 are higher. Without wishing to be bound by any particular theory, the present inventors believe that the C19-A3 peptide and glucose-C2 ligands outcompete the glutathione ligands during the self-assembly reaction forming the nanoparticles. This may be, at least in part, attributable to the relatively more steric hindrance of the thiol on the glutathione molecule than on the C19-A3 peptide ligand and the glucose-C2 ligand.

### Example 8 - Gold nanoparticle skin distribution - diameter comparison

Studies have been performed using gold NP complexes that are covalently linked to the autoantigen peptide, C19-A3. Four hours after a 50µl injection the gold NPs were distributed extensively in the papillary and reticular dermis. However, there was also evidence of gold NPs in the viable epidermis, both surrounding the site of microneedle injection and also in proximal intact areas of the skin (Figure 21A, B and C). Larger colloidal gold NPs (50 nm diameter) were injected in order to investigate whether nanoparticle size plays a role in nanoparticle distribution and cellular uptake. Analysis of skin sections showed that 50 nm Au NPs were distributed mainly in dermis near collagen and elastin fibres (Figure 22A, B and C). These results confirm that nanoparticle diameter does influence tissue distribution and cellular uptake. Nanoparticles of the present invention exhibited skin tissue distribution that is expected to be more favourable for uptake by the target dendritic cells.

### Example 9 - Generation of human recombinant IL-10 gold nanoparticles and optimisation of binding efficiency

As described above, in Example 3 and Table 17, rIL-10 incorporation efficiency of 82% has been achieved. Further work has been carried out in order to optimise the binding efficiency of rIL-10 to 3% C19-A3 GNPs. It has surprisingly been found that 100% binding of rIL-10 to 3% C19-A3 GNP (with 5% glucose C2) is achieved. This has significant advantages, not least owing to the relatively high cost of rIL-10 and the consequent highly desirable goal of minimising wastage. In brief, this involved dissolving the stock rIL-10 (0.1 mg) in 350 µl rather than 100 µl and adjustment of the ratio of GNP:rIL-10 volumes used in the binding reaction.

### Summary

The aims of these experiments were to electrostatically bind human recombinant interleukin 10 (rIL-10) to human proinsulin C19-A3 peptide (3%) and human glutamic acid decarboxylase (GAD) monomer (3%) conjugated glucose C2 GNP for *in vivo* murine studies. Ligand percentages are as determined by the input proportions of the ligand reactants during nanoparticle synthesis.

**Methods.** Human rIL-10 (100 µg) was reconstituted in water and then dialysed into sodium acetate-acetic acid buffer (0.05 M, pH 4.6). C19-A3 (3%) and GAD (3%) conjugated glucose C2 GNP (5%) were electrostatically bound to dialysed human rIL-10. Following electrostatic binding of rIL-10, the rIL-10-peptide GNPs were resuspended in sodium borate buffer (pH 8.5, 0.05 M). The size and zeta potential of rIL-10 GNP were determined using the Malvern Zetasizer, while incorporation of rIL-10 into the GNP was determined using HPLC.
**Results & conclusions.** Human rIL-10 was incorporated with 100% efficiency into 3% C19-A3-GNP (213 µg rIL-10/ml) and 3% GAD-GNP (255 µg rIL-10/ml), with no rIL-10 GNP remaining in the supernatants. DLS size analysis found that NP374 was 4.4 nm and NP377 was 8.7 nm. The zeta potential of rIL-10-C19A3-GNP (NP374) and rIL-10-GAD-GNP (NP377) were both less negatively charged (-9.6 and -18.2 mV, respectively) compared to peptide-GNP, without IL-10.

### Methods

### A) Microdialysis of rIL-10 (0.1 mg) using Pur-A-lyser Midi 3500 microdialyzer

Human rIL-10 (0.1 mg) (Cell Guidance Systems) was reconstituted with 350 µl water and stored at -20°C. An aliquot of the pre-dialysed human rIL-10 (1.9 µg) was analysed by HPLC and served as a rIL-10 standard. The remaining human rIL-10 was pipetted into a Pur-A-lyser 3500 Midi microdialyzer tube and dialysed in 250 ml of Na acetate-acetic acid buffer (50 mM, pH 4.6) for 1 h at RT, with continuous stirring. To determine whether the rIL-10 was successfully dialysed into pH 4.6 buffer, the pH of the dialysed rIL-10 was determined using a pH litmus strip (using 0.1 µl (0.028 µg) of dialysed rIL-10). A colour change from red to yellow confirmed that the pH of the dialysed rIL-10 was pH 4.6. An aliquot of post dialysed rIL-10 was analysed by HPLC (10 µl post dialysed rIL-10 mixed with 20 µl water, of which 20 µl was injected). To determine residual loss of human rIL-10 in the original vial that had contained 100 µg rIL-10 and in the dialyzer tube, TFA (50 µl) was added to each vial. From this, 40 µl samples were injected onto the HPLC column.

### HPLC chromatogram of pre dialysed human rIL-10 (1.6 µg)

An aliquot of pre-dialysed rIL-10 (10 µl, µg) was diluted with 20 µl H₂O, of which 20 µl (1.9 µg) was injected onto the HPLC column.

| **rIL-10** | **Time [Min]** | **Height [mAU]** | **Area [mAU.Min]** | **Area %** |
|---|---|---|---|---|
| **1.9 µg** | 7.93 | 197.4 | 16.318 | 100.000 |

### Original vial rinsed with TFA

To determine whether any human rIL-10 remained in the original vial that had contained 100 µg rIL-10, TFA (50 µl) was added to vial. From this, a 40 µl sample was injected onto the HPLC column.

| | **Time [Min]** | **Height [mAU]** | **Area [mAU.Min]** | **Area %** |
|---|---|---|---|---|
| **Peak 1** | 7.97 | 78.7 | 7.193 | 100.000 |

rIL-10 standard 1.9 µg = 16.318 mAu.min
Area of original tube rinsed in TFA (40 µl) = 7.193 mAu.min i.e. 0.836 µg. Therefore, total loss in 50 µl TFA = 1.04 µg.

### Dialysis tube rinsed with TFA after dialysis of human rIL-10

To check whether there was any loss of human rIL-10 post dialysis due to sticking to the dialysis membrane, the dialysis tube was rinsed with 50 µl TFA. From this, a 40 µl aliquot was injected onto the HPLC column.

| | **Time [Min]** | **Height [mAU]** | **Area [mAU.Min]** | **Area %** |
|---|---|---|---|---|
| **Peak 1** | 8.00 | 2.6 | 0.553 | 100.000 |

rIL-10 standard 1.9 µg = 16.318 mAu.min
Area of post-tube rinsed in TFA (40 µl) = 0.553 mAu.min i.e. 0.067 µg. Therefore, total loss in 50 µl TFA = 0.064 µg.

Total loss of rIL-10 due to sticking to the original vial and dialysis tube = 1.104 µg.

### Post-dialyzed human rIL-10

The volume of human rIL-10 recovered after dialysis was 349.3 µl i.e. theoretically 97 µg rIL-10 (100 µg - 1.104 µg loss - 1.9 µg pre IL-10 - 0.03 µg for pH test). An aliquot of post-dialysed human rIL-10 (10 µl) was diluted with 20 µl H₂O, of which 20 µl was injected onto the HPLC).

### HPLC chromatogram of post dialysed human rIL-10

| | **Time [Min]** | **Height [mAU]** | **Area [mAU.Min]** | **Area %** |
|---|---|---|---|---|
| **Peak 1** | 7.94 | 152.8 | 13.357 | 100.000 |

rIL-10 standard 1.9 µg = 16.318 mAu.min

Area of post-dialysed rIL-10 = 13.357 mAu.min. Therefore 82% of the rIL-10 standard i.e. 1.56 µg in 6.66 µl. Therefore, 84.3% (81.8 µg) of human rIL-10 was recovered after dialysis into Na acetate-acetic acid buffer (50 mM, pH 4.6) (from 97 µg recovered in 349.3 µl).

### B) Electrostatic binding of rIL-10 (in pH 4.6 buffer) to GNP

Dialysed human rIL-10 was incubated with GAD (3%) monomer or C19-A3 (3%) peptide conjugated glucose GNP as shown in Table 1 below.

Volumes used for electrostatic binding of dialysed rIL-10 to peptide conjugated GNP

| **Peptide-NP ID** | **Peptide** | **Peptide-GNP (µl)** | **Dialysed rIL-10 (µl)** | **Dialysed rIL-10 (µg)** | **rIL-10+peptide-NP new ID** |
|---|---|---|---|---|---|
| 67/109/1 | C19A3 | 50 | 174.6 | 41 | NP374 |
| NP235 | GAD | 50 | 174.6 | 41 | NP377 |

The samples were incubated for 1 h at RT with continuous mixing on a rocker. After 1 h, the samples were centrifuged for 3 min at 5000 rpm and checked for pellet formation. The supernatant was removed and the NP pellets were resuspended in 0.05 M Na borate buffer pH 8.5 (200 µl each).

### RESULTS AND DISCUSSION

### Gold analysis

A gold assay was performed using our standard colorimetric assay to determine the gold concentration in the nanoparticle solutions. The gold content of the rIL-10-peptide GNP batches and their supernatants are reported below.

| **Nanoparticle** | **Au (mg/ml)** |
|---|---|
| **NP374** | 0.302 |
| **NP377** | 0.287 |
| **NP374 supernatant** | 0.203 |
| **NP377 supernatant** | 0 |

### HPLC analysis

HPLC was performed using the Varian 900-LC, with a reverse phase C18 column (Acquisition time: 11.2 min; Temperature = 35°C; Wavelength 212 nm and 400 nm; Slit width = 2 nm; 95% water, 5% acetonitrile gradient (1 ml/min). At 8 min, switch to 20% water, 80% acetonitrile. To determine the amount of rIL-10 electrostatically bound to the GNP, 10 µl of NP was diluted with 140 µl TFA, of which 20 µl was injected onto the HPLC column. The supernatants were also analysed by HPLC by injecting 20 µl of supernatant, to determine the % rIL-10 remaining in the supernatant. HPLC chromatograms were acquired at 212 nm and 400 nm.

HPLC results summary using TFA (0.1%) to release rIL-10 from the peptide-GNP

| **Batch** | **µg rIL-10 bound to peptide-GNP** | **ug rIL-10 /ml GNP** | **No rIL-10/GNP** | **µg C19A3/ml rIL-10 GNP** |
|---|---|---|---|---|
| NP374 | 42.6 | 213 | 0.85 | 73.07 |
| NP377 | 51 | 255 | 1.05 | 21.31 |

NP374 (rIL-10 + 3% C19A3 GNP (67/109/1)) resuspended in 0.05 M Na borate buffer (pH 8.5)

### a) NP374 at 212 nm

| **NP374 212 nm** | **Time [Min]** | **Height [mAU]** | **Area [mAU.Min]** | **Area % [%]** |
|---|---|---|---|---|
| Peak 1 | 8.02 | 13.6 | 2.431 | 100.000 |

Peak for rIL-10 released (2.431 mAu.min) was 14.9% of the 1.9 µg human rIL-10 standard (16.318 mAU.min).

Therefore, 0.283 µg rIL-10 was released from 1.33 µl NP374.

Vol. NP374= 200 µl. Therefore, 42.6 µg rIL-10 was incorporated i.e. 213 µg/ml.

### % rIL-10 incorporation to NP374 = 100%

### Number of rIL-10 per NP

213 µg rIL-10/ (16,600 mol.wt) = 0.0128 moles
Gold content of NP377 = 0.302 mg/ml = 1.5 pmoles
12.8 nmoles rIL-10/ 15 nmol NP

Therefore, theoretically NP374 has 0.85 i.e. ~ 1 rIL-10 bound per nanoparticle

### b) NP374 at 400 nm

### HPLC chromatogram of the supernatant of NP374 (rIL-10 + NP233)

### a) NP374 supernatant at 212 nm

No peak was observed for rIL-10 confirming that the rIL-10 was all in the AuNP pellet.

### b) NP377 supernatant at 400 nm

At 400 nm, a large peak was observed for unbound C19A3 GNP. No peak was observed for rIL-10 in the supernatant, confirming that all the rIL-10-C19A3-GNP were in the pellet.

NP377 (rIL-10 + 3% GAD monomer GNP (NP235)) resuspended in 0.05 M Na borate buffer (pH 8.5)

### a) NP377 at 212 nm

| | **Time [Min]** | **Height [mAU]** | **Area [mAU.Min]** | **Area %** |
|---|---|---|---|---|
| **Peak 1** | 8.05 | 9.3 | 2.925 | 100.000 |

Peak for rIL-10 released (2.925 mAu.min) was 17.9% of the 1.9 µg human rIL-10 standard (16.318 mAU.min).

Therefore, 0.34 µg rIL-10 was released from 1.33 µl NP377.

Vol. NP377= 200 µl. Therefore, 51 µg rIL-10 was incorporated i.e. 255 µg/ml

### % rIL-10 incorporation to NP377 = 100%

### Number of rIL-10 per NP

255 µg rIL-10/ (16,600 mol.wt) = 0.0154 moles
Gold content of NP377 = 0.287 mg/ml = 1.46 pmoles
15.4 nmoles rIL-10/ 14.6 nmol NP

Therefore, theoretically NP377 has 1 rIL-10 bound per nanoparticle

### b) NP377 at 400 nm

HPLC chromatogram of the supernatant of NP377 (rIL-10 + NP234)

### a) NP377 supernatant at 212 nm

Supernatant (20 µl) was injected onto the HPLC column.

No peak was observed for rIL-10 confirming that the rIL-10 was in the AuNP pellet.

### b) NP377 supernatant at 400 nm

At 400 nm, no peak was observed for rIL-10 in the supernatant, confirming that all the rIL-10-GNP were in the pellet.

### 3.3 Zeta potential

Zeta potential measurements were performed using a ZetaNanosizer (MALVERN). The NP sample (30 µl) was dissolved in filtered water (770 µl) (0.02 µm filter). Five measurements were run for each sample and the value was given as an average zeta potential (mV).

| | **Zeta potential (mV) in water** |
|---|---|
| **NP374** | -9.6 |
| **NP377** | -18.2 |
| **NP374 supernatant** | -11.8 |
| **NP377 supernatant** | +15.4 |

| | |
|---|---|
| Zeta potential (mV) batch NP374: -9.6 ± 2.6 Zeta potential (mV) batch NP377: -18.2 ± 3.1 Zeta potential (mV) batch NP374 supernatant: -11.8 ± 3.8 Zeta potential (mV) batch NP377 supernatant: +15.4 ± 11.3 | |

### DLS nanoparticle size

DLS measurements were performed using a ZetaNanosizer (MALVERN). The NP sample (5 µl) was dissolved in filtered PBS (x10 dilution) (95 µl) (0.02 µm filter). Three measurements were run for each sample and the value was given as an average diameter by number distribution graph.

| **rIL-10-peptide GNP** | **Size (nm) in PBS (x10 diluted)** |
|---|---|
| **NP374** | 4.4 |
| **NP377** | 8.7 |

NP374 in filtered PBS (x10 dilution)
   Size (by number): 4.4 ± 1.6 nm
NP374 in filtered PBS (x10 dilution)
Size (by volume): 5.0 ± 1.8 nm
NP377 in filtered PBS (x10 dilution)
   Size (by number): 8.7 ± 5.8 nm
NP377 in filtered PBS (x10 dilution)
   Size (by volume): 15.1 ± 7.6 nm

### CONCLUSIONS

HPLC analysis of rIL-10 release from the C19A3-GNP showed that there were ~1 rIL-10 per peptide-GNP for both NP374 and NP377. The DLS nanoparticle size of NP374 and NP377 was 4.4 nm and 8.7 nm while zeta potential was -9.6 mV and -18.2 mV, respectively. Analysis of the supernatants found that the zeta potential of NP377 was positively charged (+15.4 mV) and there was minimal gold C19A3 GNP in the supernatant, indicating that the rIL-10 had bound to all the gold in the reaction. For the C19A3 GNP, as these were 2.4 fold more concentrated in terms of gold content, there was unbound C19A3-GNP remaining in the supernatant.

The specific embodiments described herein are offered by way of example, not by way of limitation. Any sub-titles herein are included for convenience only, and are not to be construed as limiting the disclosure in any way.

## Claims

1. A nanoparticle comprising:
a core comprising metal atoms;
a corona comprising a plurality of ligands covalently linked to the core, wherein said plurality of ligands comprises:
at least one carbohydrate ligand;
at least one glutathione ligand; and
at least one autoantigen peptide ligand, and wherein the plurality of ligands are in the following proportions by number:
(i) as determined by the input proportions of the ligand reactants during nanoparticle synthesis:
| | |
|---|---|
| said autoantigen peptide ligand: | 0.1 - 10% |
| said carbohydrate ligand: | 1 - 10% |
| said glutathione ligand: | 80 - 98.9%; |
and/or
(ii) as determined by NMR carried out on the
nanoparticle:
| | |
|---|---|
| said autoantigen peptide ligand: | 2 - 75% |
| said carbohydrate ligand: | 4 - 35% |
| said glutathione ligand: | 20 - 88%, |
wherein said autoantigen peptide ligand comprises a peptide selected from the group consisting of:
proinsulin peptide C19-A3 having the amino acid sequence GSLQPLALEGSLQKRGIV (SEQ ID NO: 1);
human proinsulin protein having the amino acid sequence MALWMRLLPLLALLALWGPDPAAAFVNQHLCGSHLVEALYLVCGERGFFYTPKTRREAED LQVGQVELGGGPGAGSLQPLALEGSLQKRGIVEQCCTSICSLYQLENYCN (SEQ ID NO: 7) or a variant thereof differing by addition, substitution or deletion of not more than 5 amino acids;
Glutamic acid decarboxylase (GAD) (339-352) having the amino acid sequence TVYGAFDPLLAVAD (SEQ ID NO: 2);
BDC-2.5 mimotope peptide having the amino acid sequence YVRPLWVRME (SEQ ID NO: 3);
WE-14 peptide from chromogranin A 342-355 having the amino acid sequence WSRMDQLAKELTAE (SEQ ID NO: 4);
Insulin B (9-23) having the amino acid sequence SHLVEALYLVSGERG (SEQ ID NO: 5); and
a HIB peptide having the amino acid sequence DLQTLALWSRMD (SEQ ID NO: 12),
or a hybrid peptide comprising two or more of said peptides.

2. The nanoparticle according to claim 1, wherein the plurality of ligands are in the following proportions by number, as determined by NMR carried out on the nanoparticle:
| | |
|---|---|
| said autoantigen peptide ligand: | 4 - 15% |
| said carbohydrate ligand: | 12 - 35% |
| said glutathione ligand: | 50 - 88%. |

3. The nanoparticle according to claim 1 or claim 2, wherein the nanoparticle further comprises an anti-inflammatory cytokine that is non-covalently bound to the corona of the nanoparticle, wherein the anti-inflammatory cytokine is optionally selected from the group consisting of: IL-10, TGF-beta 1 and TGF-beta 2.

4. A nanoparticle comprising:
a core comprising metal atoms;
a corona comprising a plurality of ligands covalently linked to the core, wherein said plurality of ligands comprises at least one carbohydrate ligand and at least one glutathione ligand; and
at least one IL-10 polypeptide non-covalently bound to the corona.

5. A pharmaceutical composition comprising a plurality of nanoparticles as defined in any one of the preceding claims and a pharmaceutically acceptable carrier, salt and/or diluent, optionally wherein the composition is in the form of a vaccine.

6. The pharmaceutical composition according to claim 5, wherein the plurality of nanoparticles comprises proinsulin C19-A3 peptide-carrying nanoparticles and IL-10-carrying nanoparticles, and wherein said C19-A3 peptide-carrying nanoparticles and IL-10-carrying nanoparticles are present at a ratio such that the ratio of C19-A3 peptide and IL-10 polypeptide is between 1:1 and 1:100 IL-10:C19-A3, by number.

7. A nanoparticle according to any one of claims 1 to 4 or a pharmaceutical composition according to claim 5 or claim 6 for use in medicine.

8. A nanoparticle for use in antigen specific immunotherapy of an autoimmune disorder in a mammalian subject, wherein said nanoparticle comprises:
a core comprising metal atoms;
a corona comprising a plurality of ligands covalently linked to the core, wherein said plurality of ligands comprises:
at least one carbohydrate ligand;
at least one glutathione ligand; and
at least one autoantigen peptide ligand,
and wherein the plurality of ligands are in the following proportions by number:
(i) as determined by the input proportions of the ligand reactants during nanoparticle synthesis:
| | |
|---|---|
| said autoantigen peptide ligand: | 0.1 - 10% |
| said carbohydrate ligand: | 1 - 10% |
| said glutathione ligand: | 80 - 98.9%; |
and/or
(ii) as determined by NMR carried out on the
nanoparticle:
| | |
|---|---|
| said autoantigen peptide ligand: | 2 - 75% |
| said carbohydrate ligand: | 4 - 35% |
| said glutathione ligand: | 20 - 88% |
wherein the nanoparticle:
(a) delivers the autoantigen peptide to antigen presenting cells (APCs); and/or
(b) delivers the autoantigen peptide to draining lymph nodes via migratory DCs; and/or
(c) delivers the autoantigen peptide to pancreatic lymph nodes.

9. A nanoparticle or pharmaceutical composition for use in antigen specific immunotherapy of an autoimmune disorder in a mammalian subject, wherein the nanoparticle is as defined in any one of claims 1 to 4 and wherein the pharmaceutical composition is as defined in claim 5 or claim 6.

10. The nanoparticle or composition for use according to claim 8 or claim 9, wherein said autoimmune disorder is diabetes mellitus type 1.

11. The nanoparticle or composition for use according to any one of claims 8 to 10, wherein IL-10-carrying nanoparticles or composition thereof and C19-A3 peptide-carrying nanoparticles or composition thereof are delivered simultaneously or sequentially.

12. The nanoparticle or composition for use according to any one of claims 8 to 11, wherein the nanoparticle or composition thereof is for:
inducing a tolerogenic phenotype in a dendritic cell (DC);
enhancing production of tolerogenic cytokines;
promoting regulatory T cell generation; and/or
preserving pancreatic beta cell function,
in said subject.

13. The nanoparticle or composition for use according to any one of claims 8 to 12, wherein the nanoparticle or composition thereof:
(a) is delivered via a dermal route of administration; and/or
(b) is delivered via microneedle injection; and/or
(c) delivers the autoantigen peptide and/or the anti-inflammatory cytokine to antigen presenting cells (APCs); and/or
(d) delivers the autoantigen peptide and/or the anti-inflammatory cytokine to draining lymph nodes via migratory DCs; and/or
(e) delivers the autoantigen peptide and/or the anti-inflammatory cytokine to pancreatic lymph nodes.

14. The nanoparticle or composition for use according to any one of claims 8 to 13, wherein the subject:
(a) has been diagnosed with, or is at risk of developing, diabetes mellitus type 1 or pre-diabetes; and optionally
(b) has been tested positive for one or more autoantibodies associated with diabetes mellitus type 1.

15. A method for producing a nanoparticle as defined in any one of claims 1 to 4, comprising:
providing at least one thiol-derivatised autoantigen peptide, at least one glutathione, at least one thiol-derivatised carbohydrate, at least one salt of a core-forming metal, and a reducing agent;
reacting the at least one thiol-derivatised autoantigen peptide, the at least one glutathione, the at least one thiol-derivatised carbohydrate, the at least one salt of a core-forming metal, and the reducing agent so that during self-assembly of the nanoparticle, the nanoparticle core attaches the autoantigen peptide, the glutathione and the carbohydrate via their respective linkers.

## Patentansprüche

1. Nanoteilchen, das Folgendes umfasst:
einen Kern, der Metallatome umfasst;
eine Hülle, die eine Vielzahl von Liganden umfasst, die kovalent an den Kern gebunden sind, wobei die Vielzahl von Liganden folgende umfasst:
zumindest einen Kohlehydratliganden;
zumindest einen Glutathionliganden und
zumindest einen Autoantigenpeptidliganden,
und wobei die Vielzahl von Liganden in folgenden zahlenmäßigen Anteilen vorliegen:
(i) wie mittels Eingabeanteilen der Ligandenreaktanten während der Nanoteilchensynthese bestimmt:
| | |
|---|---|
| Autoantigen peptid ligand: | 0,1 bis 10 % |
| Kohlenhydratligand: | 1 bis 10 % |
| Glutathionligand: | 80 bis 98,9 % |
und/oder
(ii) wie mittels in Bezug auf das Nanoteilchen durchgeführter NMR bestimmt:
| | |
|---|---|
| Autoantigen peptid ligand: | 2 bis 75 % |
| Kohlenhydratligand: | 4 bis 35 % |
| Glutathionligand: | 20 bis 88 % |
wobei der Autoantigenpeptidligand ein Peptid umfasst, das aus der aus folgenden bestehenden Gruppe ausgewählt ist:
Proinsulinpeptid C19-A3 mit der Aminosäuresequenz GSLQPLALEGSLQKRGIV (SEQ ID NO: 1);
menschlichem Proinsulinprotein mit der Aminosäuresequenz oder einer Variante davon, die sich durch Addition, Substitution oder Deletion von nicht mehr als 5 Aminosäuren unterscheidet;
Glutaminsäuredecarboxylase (GAD) (339-352) mit der Aminosäuresequenz TVYGAFDPLLAVAD (SEQ ID NO: 2);
BDC-2.5-Mimotoppeptid mit der Aminosäuresequenz YVRPLWVRME (SEQ ID NO: 3);
WE-14-Peptid von Chromogranin A 342-355 mit der Aminosäuresequenz WSRMDQLAKELTAE (SEQ ID NO: 4);
Insulin B (9-23) mit der Aminosäuresequenz SHLVEALYLVSGERG (SEQ ID NO: 5) und
einem HIB-Peptid mit der Aminosäuresequenz DLQTLALWSRMD (SEQ ID NO: 12)
oder einem Hybridpeptid, das zwei oder mehr dieser Peptide umfasst.

2. Nanoteilchen nach Anspruch 1, wobei die Vielzahl von Liganden in folgenden zahlenmäßigen Anteilen vorliegen, wie mittels in Bezug auf das Nanoteilchen durchgeführter NMR ermittelt:
| | |
|---|---|
| Autoantigen peptid ligand: | 4 bis 15 % |
| Kohlenhydratligand: | 12 bis 35 % |
| Glutathionligand: | 50 bis 88 % |

3. Nanoteilchen nach Anspruch 1 oder 2, wobei das Nanoteilchen ferner ein entzündungshemmendes Zytokin umfasst, das nicht-kovalent an die Hülle des Nanoteilchens gebunden ist, wobei das entzündungshemmende Zytokin gegebenenfalls aus der aus IL-10, TGF-β 1 und TGF-β 2 bestehenden Gruppe ausgewählt ist.

4. Nanoteilchen, das Folgendes umfasst:
einen Kern, der Metallatome umfasst;
eine Hülle, die eine Vielzahl von Liganden umfasst, die kovalent an den Kern gebunden sind, wobei die Vielzahl von Liganden zumindest einen Kohlenhydratliganden und zumindest einen Glutathionliganden umfasst; und
zumindest ein IL-10-Polypeptid, das nicht-kovalent an die Hülle gebunden ist.

5. Pharmazeutische Zusammensetzung, die eine Vielzahl von Nanoteilchen wie in einem der vorangegangenen Ansprüche definiert und einen pharmazeutisch annehmbaren Träger, ein pharmazeutisch annehmbares Salz und/oder ein pharmazeutisch annehmbares Verdünnungsmittel umfasst, wobei die Zusammensetzung gegebenenfalls in Form einer Vakzine vorliegt.

6. Pharmazeutische Zusammensetzung nach Anspruch 5, wobei die Vielzahl von Nanoteilchen Proinsulin-C19-A3-Peptid-tragende Nanoteilchen und IL-10-tragende Nanoteilchen umfasst und wobei die C19-A3-Peptid-tragenden Nanoteilchen und die IL-10-tragenden Nanoteilchen in einem solchen Verhältnis vorliegen, dass das zahlenmäßige Verhältnis von C19-A3-Peptid zu IL-10-Polypeptid zwischen 1:1 und 1:100 IL-10:C19-A3 liegt.

7. Nanoteilchen nach einem der Ansprüche 1 bis 4 oder pharmazeutische Zusammensetzung nach Anspruch 5 oder 6 zur Verwendung in einem Medikament.

8. Nanoteilchen zur Verwendung in antigenspezifischer Immuntherapie einer Autoimmunerkrankung in einem Säugetierindividuum, wobei das Nanoteilchen Folgendes umfasst:
einen Kern, der Metallatome umfasst;
eine Hülle, die eine Vielzahl von Liganden umfasst, die kovalent an den Kern gebunden sind, wobei die Vielzahl von Liganden folgende umfasst:
zumindest einen Kohlenhydratliganden;
zumindest einen Glutathionliganden und
zumindest einen Autoantigenpeptidliganden,
und wobei die Vielzahl von Liganden in folgenden zahlenmäßigen Anteilen vorliegen:
(i) wie mittels Eingabeanteilen der Ligandenreaktanten während der Nanoteilchensynthese bestimmt:
| | |
|---|---|
| Autoantigen peptid ligand: | 0,1 bis 10 % |
| Kohlenhydratligand: | 1 bis 10 % |
| Glutathionligand: | 80 bis 98,9 % |
und/oder
(ii) wie mittels in Bezug auf das Nanoteilchen durchgeführter NMR bestimmt:
| | |
|---|---|
| Autoantigen peptid ligand: | 2 bis 75 % |
| Kohlenhydratligand: | 4 bis 35 % |
| Glutathionligand: | 20 bis 88 % |
wobei das Nanoteilchen:
(a) das Autoantigenpeptid zu Antigen-präsentierenden Zellen (APC) zuführt; und/oder
(b) das Autoantigenpeptid mittels migrierender DC zu drainierenden Lymphknoten zuführt; und/oder
(c) das Autoantigenpeptid zu Pankreaslymphknoten zuführt.

9. Nanoteilchen oder pharmazeutische Zusammensetzung zur Verwendung in antigenspezifischer Immuntherapie einer Autoimmunerkrankung bei einem Säugetierindividuum, wobei das Nanoteilchen wie in einem der Ansprüche 1 bis 4 definiert ist und wobei die pharmazeutische Zusammensetzung wie in Anspruch 5 oder 6 definiert ist.

10. Nanoteilchen oder Zusammensetzung zur Verwendung nach Anspruch 8 oder 9, wobei die Autoimmunerkrankung Diabetes mellitus Typ 1 ist.

11. Nanoteilchen oder Zusammensetzung zur Verwendung nach einem der Ansprüche 8 bis 10, wobei IL-10-tragende Nanoteilchen oder eine Zusammensetzung davon und C19-A3-Peptid-tragende Nanoteilchen oder eine Zusammensetzung davon gleichzeitig oder nacheinander zugeführt werden.

12. Nanoteilchen oder Zusammensetzung zur Verwendung nach einem der Ansprüche 8 bis 11, wobei das Nanoteilchen oder die Zusammensetzung davon dazu dient, in dem Individuum:
einen tolerogenen Phänotyp in einer dendritischen Zelle (DC) zu induzieren;
die Produktion von tolerogenen Zytokinen zu steigern;
die Erzeugung von regulatorischen T-Zellen zu fördern und/oder
die Funktion von β-Zellen in der Bauchspeicheldrüse zu erhalten.

13. Nanoteilchen oder Zusammensetzung zur Verwendung nach einem der Ansprüche 8 bis 12, wobei das Nanoteilchen oder die Zusammensetzung davon:
(a) über einen dermalen Verabreichungsweg zugeführt wird; und/oder
(b) mittels Mikronadelinjektion zugeführt wird; und/oder
(c) das Autoantigenpeptid und/oder das entzündungshemmende Zytokin zu Antigen-präsentierenden Zellen (APC) zuführt; und/oder
(d) das Autoantigenpeptid und/oder das entzündungshemmende Zytokin zu drainierenden Lymphknoten mittels migrierender DC zuführt; und/oder
(e) das Autoantigenpeptid und/oder das entzündungshemmende Zytokin zu Pankreaslymphknoten zuführt.

14. Nanoteilchen oder Zusammensetzung zur Verwendung nach einem der Ansprüche 8 bis 13, wobei:
(a) bei dem Individuum Diabetes mellitus Typ 1 oder Prädiabetes diagnostiziert wurde oder das Risiko besteht, dass es an Diabetes mellitus Typ 1 oder Prädiabetes erkrankt; und gegebenenfalls
(b) das Individuum positiv in Bezug auf einen oder mehrere Autoantikörper getestet wurde, die mit Diabetes mellitus Typ 1 in Zusammenhang stehen.

15. Verfahren zur Produktion eines Nanoteilchens nach einem der Ansprüche 1 bis 4, wobei das Verfahren Folgendes umfasst:
das Bereitstellen zumindest eines Thiol-derivatisierten Autoantigenpeptids, zumindest eines Glutathions, zumindest eines Thiol-derivatisierten Kohlehydrats, zumindest eines Salzes eines kernbildenden Metalls und eines Reduktionsmittels;
das Umsetzen des zumindest einen Thiol-derivatisierten Autoantigenpeptids, des zumindest einen Glutathions, des zumindest einen Thiol-derivatisierten Kohlenhydrats, des zumindest einen Salzes eines kernbildenden Metalls und des Reduktionsmittels, so dass während der Selbstassemblierung des Nanoteilchens der Nanoteilchenkern das Autoantigenpeptid, das Glutathion und das Kohlenhydrat über ihre entsprechenden Linker bindet.

## Revendications

1. Nanoparticule comprenant :
un noyau comprenant des atomes métalliques ;
une couronne comprenant une pluralité de ligands liés de façon covalente au noyau, dans laquelle ladite pluralité de ligands comprend :
au moins un ligand glucidique ;
au moins un ligand du glutathion ; et
au moins un ligand de peptide autoantigène,
et dans laquelle la pluralité de ligands est dans les proportions suivantes en nombre :
(i) tel que déterminé par les proportions de départ des réactifs de ligand pendant la synthèse de la nanoparticule :
| | |
|---|---|
| ledit ligand de peptide autoantigène : | 0,1-10 % |
| ledit ligand glucidique : | 1-10 % |
| ledit ligand du glutathion : | 80-98,9 % ; |
et/ou
(ii) tel que déterminé par une RMN réalisée sur la nanoparticule :
| | |
|---|---|
| ledit ligand de peptide autoantigène : | 2-75 % |
| ledit ligand glucidique : | 4-35 % |
| ledit ligand du glutathion : | 20-88 %, |
dans laquelle ledit ligand de peptide autoantigène comprend un peptide sélectionné dans le groupe consistant en :
un peptide de proinsuline C19-A3 possédant la séquence d'acides aminés GSLQPLALEGSLQKRGIV (SEQ ID NO: 1) ;
une protéine de proinsuline humaine possédant la séquence d'acides aminés MALWMRLLPLLALLALWGPDPAAAFVNQHLCGSHLVEALYLVCGERGFFYTPKTRREAE DLQVGQVELGGGPGAGSLQPLALEGSLQKRGIVEQCCTSICSLYQLENYCN (SEQ ID NO: 7) ou un variant de celle-ci différant par une addition, une substitution ou une délétion de 5 acides aminés au maximum ;
une acide glutamique décarboxylase (GAD) (339-352) possédant la séquence d'acides aminés TVYGAFDPLLAVAD (SEQ ID NO: 2) ;
un peptide mimotope BDC-2.5 possédant la séquence d'acides aminés YVRPLWVRME (SEQ ID NO: 3) ;
un peptide WE-14 de la chromogranine A 342-355 possédant la séquence d'acides aminés WSRMDQLAKELTAE (SEQ ID NO: 4) ;
l'insuline B (9-23) possédant la séquence d'acides aminés SHLVEALYLVSGERG (SEQ ID NO: 5) ; et
un peptide HIB possédant la séquence d'acides aminés DLQTLALWSRMD (SEQ ID NO: 12),
ou un peptide hybride comprenant deux desdits peptides ou plus.

2. Nanoparticule selon la revendication 1, dans laquelle la pluralité de ligands est dans les proportions suivantes en nombre, tel que déterminé par une RMN réalisée sur la nanoparticule :
| | |
|---|---|
| ledit ligand de peptide autoantigène : | 4-15 % |
| ledit ligand glucidique : | 12-35 % |
| ledit ligand du glutathion : | 50-88 %. |

3. Nanoparticule selon la revendication 1 ou la revendication 2, dans laquelle la nanoparticule comprend en outre une cytokine anti-inflammatoire qui est liée de façon non covalente à la couronne de la nanoparticule, dans laquelle la cytokine anti-inflammatoire est facultativement sélectionnée dans le groupe consistant en : l'IL-10, le TGF-bêta 1 et le TGF-bêta 2.

4. Nanoparticule comprenant :
un noyau comprenant des atomes métalliques ;
une couronne comprenant une pluralité de ligands liés de façon covalente au noyau, dans laquelle ladite pluralité de ligands comprend au moins un ligand glucidique et au moins un ligand du glutathion ; et
au moins un polypeptide d'IL-10 lié de façon non covalente à la couronne.

5. Composition pharmaceutique comprenant une pluralité de nanoparticules tel que défini dans l'une quelconque des revendications précédentes et un véhicule, un sel et/ou un diluant pharmaceutiquement acceptable, facultativement dans laquelle la composition est sous la forme d'un vaccin.

6. Composition pharmaceutique selon la revendication 5, dans laquelle la pluralité de nanoparticules comprend des nanoparticules portant le peptide de proinsuline C19-A3 et des nanoparticules portant l'IL-10, et dans laquelle lesdites nanoparticules portant le peptide de proinsuline C19-A3 et nanoparticules portant l'IL-10 sont présentes à un rapport tel que le rapport de peptide C19-A3 et de polypeptide d'IL-10 est compris entre 1:1 et 1:100 d'IL-10:C19-A3 en nombre.

7. Nanoparticule selon l'une quelconque des revendications 1 à 4 ou composition pharmaceutique selon la revendication 5 ou la revendication 6 destinée à être utilisée en médecine.

8. Nanoparticule destinée à être utilisée dans une immunothérapie spécifique d'antigènes d'un trouble auto-immun chez un sujet mammifère, où ladite nanoparticule comprend :
un noyau comprenant des atomes métalliques ;
une couronne comprenant une pluralité de ligands liés de façon covalente au noyau, dans laquelle ladite pluralité de ligands comprend :
au moins un ligand glucidique ;
au moins un ligand du glutathion ; et
au moins un ligand de peptide autoantigène, et dans laquelle la pluralité de ligands est dans les proportions suivantes en nombre :
(i) tel que déterminé par les proportions de départ des réactifs de ligand pendant la synthèse de la nanoparticule :
| | |
|---|---|
| ledit ligand de peptide autoantigène : | 0,1-10 % |
| ledit ligand glucidique : | 1-10 % |
| ledit ligand du glutathion : | 80-98,9 % ; et/ou |
(ii) tel que déterminé par une RMN réalisée sur la nanoparticule :
| | |
|---|---|
| ledit ligand de peptide autoantigène : | 2-75 % |
| ledit ligand glucidique : | 4-35 % |
| ledit ligand du glutathion : | 20-88 % |
dans laquelle la nanoparticule :
(a) délivre le peptide autoantigène aux cellules présentatrices d'antigène (APC) ; et/ou
(b) délivre le peptide autoantigène aux ganglions lymphatiques de drainage via des CD migratoires ; et/ou
(c) délivre le peptide autoantigène aux ganglions lymphatiques pancréatiques.

9. Nanoparticule ou composition pharmaceutique destinée à être utilisée dans une immunothérapie spécifique d'antigènes d'un trouble auto-immun chez un sujet mammifère, où la nanoparticule est telle que définie dans l'une quelconque des revendications 1 à 4 et où la composition pharmaceutique est telle que définie dans la revendication 5 ou la revendication 6.

10. Nanoparticule ou composition destinée à être utilisée selon la revendication 8 ou la revendication 9, où ledit trouble auto-immun est le diabète sucré de type 1.

11. Nanoparticule ou composition destinée à être utilisée selon l'une quelconque des revendications 8 à 10, dans laquelle les nanoparticules portant l'IL-10 ou la composition de celles-ci et les nanoparticules portant le peptide C19-A3 ou la composition de celles-ci sont délivrées simultanément ou séquentiellement.

12. Nanoparticule ou composition destinée à être utilisée selon l'une quelconque des revendications 8 à 11, dans laquelle la nanoparticule ou la composition de celle-ci est destinée à :
induire un phénotype tolérogène dans une cellule dendritique (CD) ;
améliorer la production de cytokines tolérogènes ;
favoriser la génération de cellules T régulatrices ; et/ou
préserver la fonction des cellules bêta pancréatiques, chez ledit sujet.

13. Nanoparticule ou composition destinée à être utilisée selon l'une quelconque des revendications 8 à 12, dans laquelle la nanoparticule ou la composition de celle-ci :
(a) est délivrée via une voie d'administration dermique ; et/ou
(b) est délivrée via une injection par micro-aiguille ; et/ou
(c) délivre le peptide autoantigène et/ou la cytokine anti-inflammatoire aux cellules présentatrices d'antigène (APC) ; et/ou
(d) délivre le peptide autoantigène et/ou la cytokine anti-inflammatoire aux ganglions lymphatiques de drainage via des CD migratoires ; et/ou
(e) délivre le peptide autoantigène et/ou la cytokine anti-inflammatoire aux ganglions lymphatiques pancréatiques.

14. Nanoparticule ou composition destinée à être utilisée selon l'une quelconque des revendications 8 à 13, dans laquelle le sujet :
(a) a été diagnostiqué comme présentant, ou étant à risque de développer, un diabète sucré de type 1 ou un pré-diabète ; et facultativement
(b) a été testé positif pour un ou plusieurs auto-anticorps associés au diabète sucré de type 1.

15. Procédé pour produire une nanoparticule telle que définie dans l'une quelconque des revendications 1 à 4, comprenant :
la mise à disposition d'au moins peptide autoantigène dérivatisé avec un thiol, d'au moins un glutathion, d'au moins un glucide dérivatisé avec un thiol, d'au moins un sel d'un métal formant un noyau, et d'un agent de réduction ;
la réaction du au moins un peptide autoantigène dérivatisé avec un thiol, du au moins un glutathion, du au moins un glucide dérivatisé avec un thiol, du au moins un sel d'un métal formant un noyau, et de l'agent de réduction de sorte que, pendant l'auto-assemblage de la nanoparticule, le noyau de la nanoparticule s'attache au peptide autoantigène, au glutathion et au glucide via leurs segments de liaison respectifs.
